# EUROPEAN PATENT SPECIFICATION

(11) **EP 3 404 041 B1**
(45) Date of publication and mention of the grant of the patent: **22.07.2020**
(21) Application number: 18182009.3
(22) Date of filing: 18.04.2012
(51) Int. Cl.: C07K 16/22

(54) **METHOD FOR TREATING OSTEOPOROSIS**
VERFAHREN ZUR BEHANDLUNG VON OSTEOPOROSE
PROCÉDÉ DE TRAITEMENT DE L'OSTÉOPOROSE

(30) Priority: 19.04.2011 US 201161477065 P
(43) Date of publication of application: 21.11.2018
(62) Divisional of application: 12717002.5
(73) Proprietor: Amgen Inc., Thousand Oaks, CA 91320-1799 (US)
(72) Inventor: SAN MARTIN, Javier, Thousand Oaks, CA 91320-1799 (US); WASSERMAN, Scott, Newbury Park, CA 91320 (US)
(74) Representative: J A Kemp LLP

(56) References cited:
- WO-A2-2006/119062
- WO-A2-2006/119107
- WO-A2-2008/115732
- US-A1- 2009 074 763
- LEWIECKI E MICHAEL: "Sclerostin monoclonal antibody therapy with AMG 785: a potential treatment for osteoporosis", EXPERT OPINION ON BIOLOGICAL THERAPY, INFORMA HEALTHCARE, UK, vol. 11, no. 1, 1 January 2011 (2011-01-01), pages 117-127, XP009160541, ISSN: 1744-7682, DOI: 10.1517/14712598.2011.540565
- TILMAN D RACHNER ET AL: "Osteoporosis: now and the future", THE LANCET, vol. 377, no. 9773, 1 April 2011 (2011-04-01), pages 1276-1287, XP055031012, ISSN: 0140-6736, DOI: 10.1016/S0140-6736(10)62349-5
- STUART L. SILVERMAN: "Sclerostin", JOURNAL OF OSTEOPOROSIS, vol. 2010, 1 January 2010 (2010-01-01), pages 1-3, XP055031014, ISSN: 2090-8059, DOI: 10.4061/2010/941419
- PAPAPOULOS SOCRATES E: "Targeting sclerostin as potential treatment of osteoporosis", ANNALS OF THE RHEUMATIC DISEASES, B M J GROUP, UK, vol. 70, no. Suppl. 1, 1 March 2011 (2011-03-01), pages I119-I122, XP009160540, ISSN: 1468-2060, DOI: 10.1136/ARD.2010.141150
- N N: "Amgen presents demosub and sclerostin antibody data at the American Society for Bone and Mineral Research Annual Meeting", INTERNET CITATION, 1 January 2006 (2006-01-01), pages 1-3, XP002517455, Retrieved from the Internet: URL:www.amgen.com/media/media_pr_detail.js p?releaseID=907028 [retrieved on 2009-02-26]
- MCCLUNG M R ET AL: "Romosozumab in postmenopausal women with low bone mineral density", NEW ENGLAND JOURNAL OF MEDICINE, THE - NEJM -, MASSACHUSETTS MEDICAL SOCIETY, US, vol. 370, no. 5, 1 January 2014 (2014-01-01), pages 412-420, XP009178243, ISSN: 0028-4793, DOI: 10.1056/NEJMOA1305224

## Description

### TECHNICAL FIELD OF THE INVENTION

The invention generally relates to methods of increasing bone mineral density and treating osteoporosis using an anti-sclerostin antibody.

### BACKGROUND OF THE INVENTION

Osteoporosis is a debilitating disease in humans and is characterized by marked decreases in skeletal bone mass and mineral density, structural deterioration of bone, including degradation of bone microarchitecture and corresponding increases in bone fragility (i.e., decreases in bone strength), and susceptibility to fracture in afflicted individuals. Osteoporosis in humans is generally preceded by clinical osteopenia, a condition found in approximately 25 million people in the United States. Another 7-8 million patients in the United States have been diagnosed with clinical osteoporosis. The frequency of osteoporosis in the human population increases with age. Among Caucasians, osteoporosis is predominant in women who, in the United States, comprise 80% of the osteoporosis patient pool. The increased fragility and susceptibility to fracture of skeletal bone in the aged is aggravated by the greater risk of accidental falls in this population. Fractured hips, wrists, and vertebrae are among the most common injuries associated with osteoporosis and low bone mineral density. Hip fractures in particular are extremely uncomfortable and expensive for the patient, and for women, correlate with high rates of mortality and morbidity.

### SUMMARY OF THE INVENTION

The invention is directed to an anti-sclerostin antibody comprising a CDR-H1 of SEQ ID NO:245, a CDR-H2 of SEQ ID NO:246, a CDR-H3 of SEQ ID NO:247, a CDR-L1 of SEQ ID NO:78, a CDR-L2 of SEQ ID NO:79 and a CDR-L3 of SEQ ID NO:80, for use in a method for increasing bone mineral density in a postmenopausal woman, the method comprising administering to a postmenopausal woman suffering from osteoporosis said anti-sclerostin antibody in an amount and for a treatment period effective to increase lumbar vertebrae bone mineral density (BMD) at least 9% from pretreatment baseline at twelve months following initial administration of the anti-sclerostin antibody; wherein the amount of anti-sclerostin antibody administered is about 140 mg or about 210 mg and the amount of the anti-sclerostin antibody is administered at an interval no more frequent than once a month.

The method may comprise administering to a postmenopausal woman with osteoporosis an anti-sclerostin antibody in an amount of from about 70 mg to about 210 mg at an interval of once a month. Optionally, the anti-sclerostin antibody is administered over a treatment period of about three months to about 18 months. In various embodiments of the disclosure, the method comprises administering to a postmenopausal woman with osteoporosis an anti-sclerostin antibody in an amount of from about 140 mg to about 210 mg at an interval of every three months, optionally, over a treatment period of about six months to about 18 months.

The use of anti-sclerostin antibodies in any of the methods disclosed herein or for preparation of medicaments for administration according to any of the methods disclosed herein is specifically contemplated by the disclosure. In this regard, the disclosure includes an anti-sclerostin antibody for use in a method of increasing bone mineral density in a postmenopausal woman, the method comprising administering to a postmenopausal woman having a lumbar vertebrae T-score of less than or equal to -2 an anti-sclerostin antibody in an amount and for a time effective to increase lumbar vertebrae bone mineral density (BMD) at least about 9% from pretreatment baseline at twelve months following initial administration of the anti-sclerostin antibody. The disclosure further includes an anti-sclerostin antibody for use in a method for treating osteoporosis, the method comprising administering to a postmenopausal woman with osteoporosis an anti-sclerostin antibody in an amount of from about 70 mg to about 210 mg at an interval of once a month for a treatment period, optionally, of about three months to about 18 months. The disclosure further includes an anti-sclerostin antibody for use in a method for treating osteoporosis, the method comprising administering to a postmenopausal woman with osteoporosis an anti-sclerostin antibody in an amount of from about 140 mg to about 210 mg at an interval of once every three months for a treatment period, optionally, of about six months to about 18 months.

In addition, the disclosure provides use of an anti-sclerostin antibody in preparation of a medicament for increasing bone mineral density in a postmenopausal woman having a lumbar vertebrae T-score of less than or equal to -2 in an amount effective to increase lumbar vertebrae bone mineral density (BMD) at least about 9% from pretreatment baseline at twelve months following initial administration of the anti-sclerostin antibody.

The disclosure also includes use of an anti-sclerostin antibody in preparation of a medicament for treating osteoporosis in a postmenopausal woman in an amount of from about 70 mg to about 210 mg administered at an interval of once a month for a treatment period, optionally, of about three months to about 18 months, as well as use of an anti-sclerostin antibody in preparation of a medicament for treating osteoporosis in an amount of from about 140 mg to about 210 mg administered at an interval of once every three months for a treatment period, optionally, of about six months to about 18 months.

The foregoing summary is not intended to define every aspect of the invention, and additional aspects are described in other sections, such as the Detailed Description. The entire document is intended to be related as a unified disclosure, and it should be understood that all combinations of features described herein are contemplated, even if the combination of features are not found together in the same sentence, or paragraph, or section of this document. With respect to aspects of the invention described or claimed with "a" or "an," it should be understood that these terms mean "one or more" unless context unambiguously requires a more restricted meaning. The term "or" should be understood to encompass items in the alternative or together, unless context unambiguously requires otherwise. If aspects of the invention are described as "comprising" a feature, embodiments also are contemplated "consisting of' or "consisting essentially of' the feature.

### BRIEF DESCRIPTION OF THE FIGURES

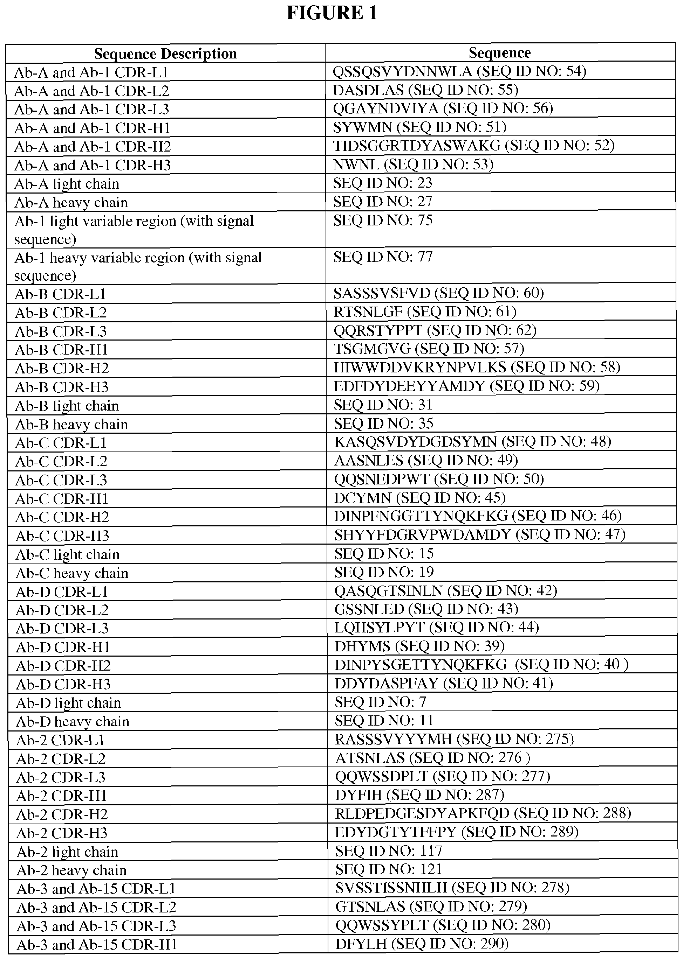

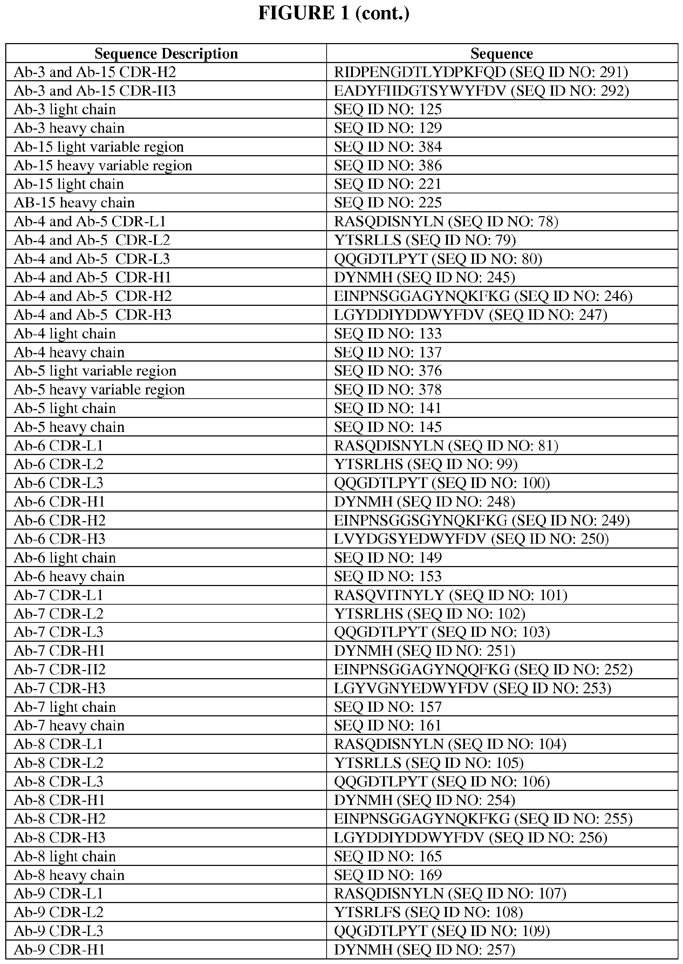

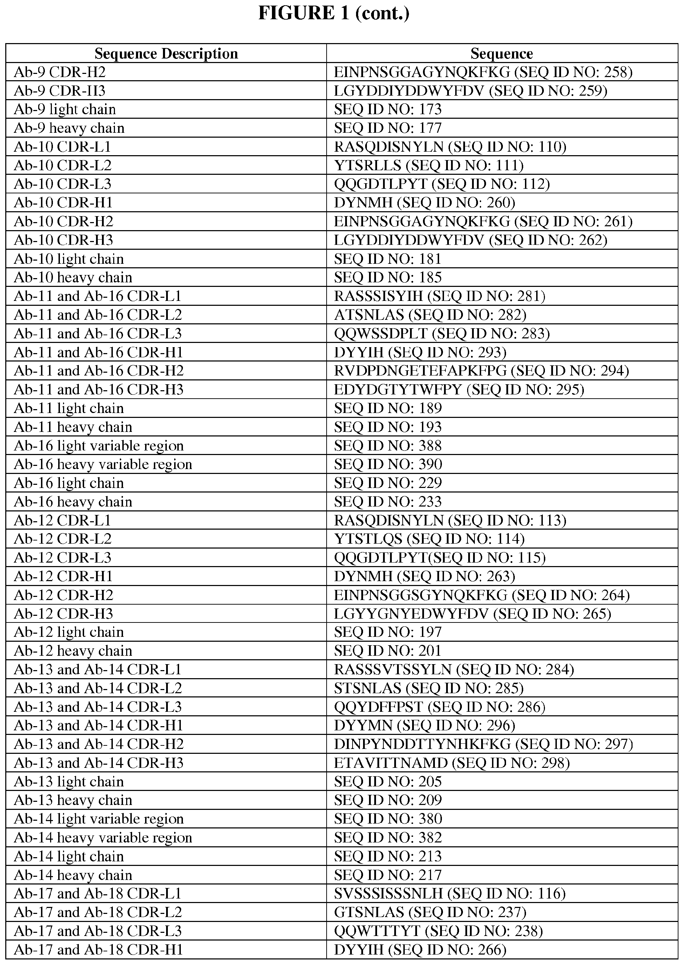

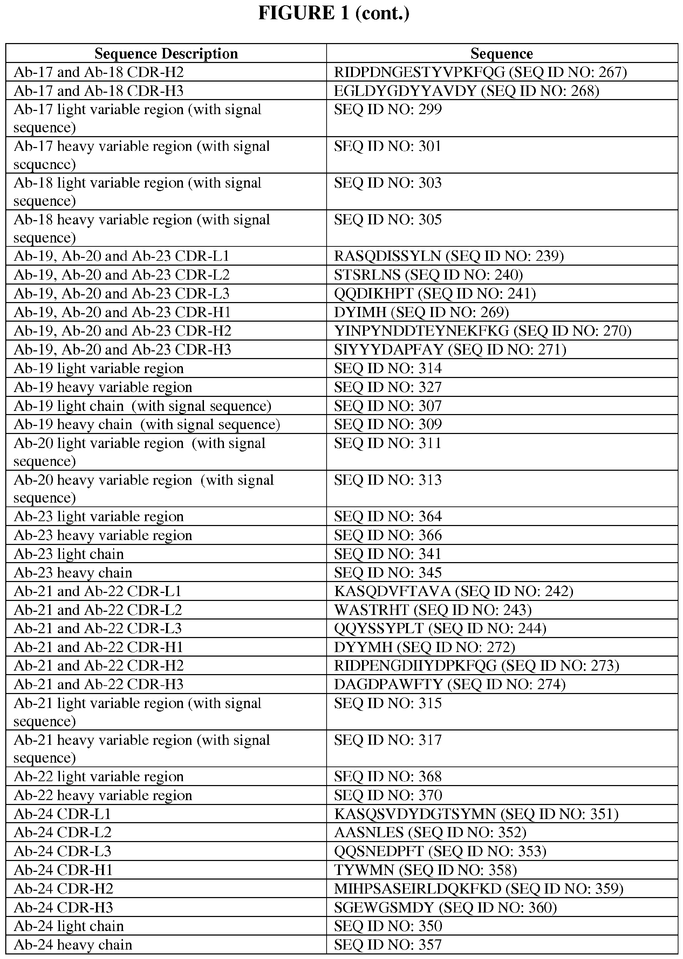

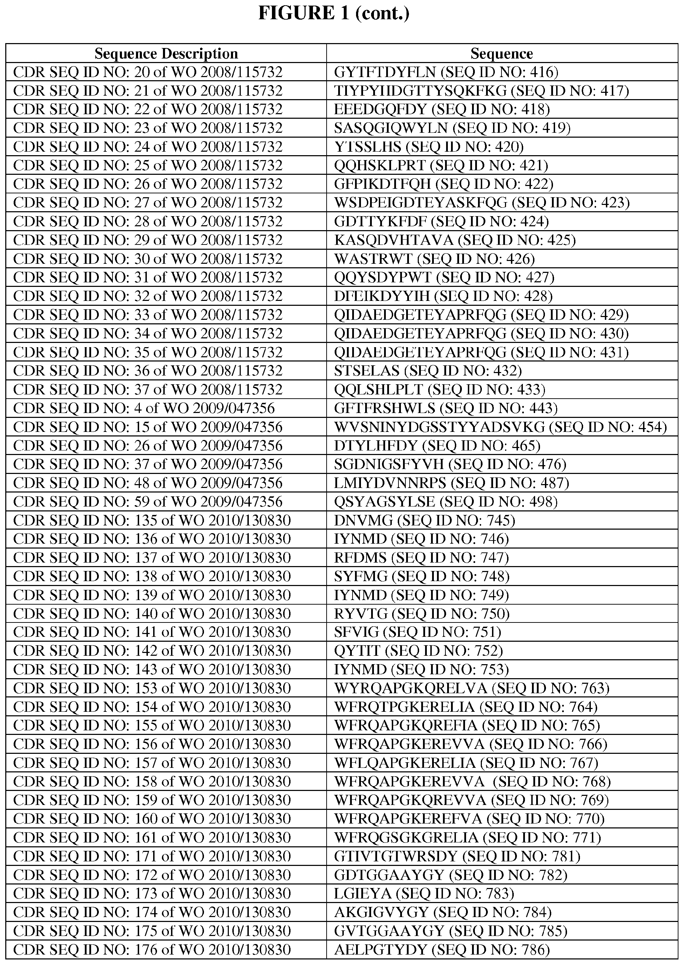

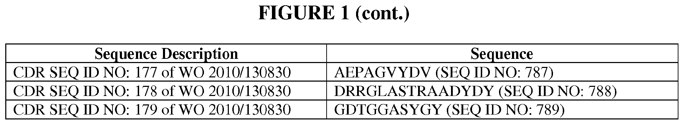

Figure 1 is a chart listing amino acid sequences and sequence identifiers for amino acid sequences of various anti-sclerostin antibodies described herein. The sequence identifiers refer to amino acid sequences provided in the Sequence Listing submitted herewith. The amino acid sequences also are set forth in U.S. Patent Publication No. 20070110747, International Patent Publication No. WO 2008/115732, International Patent Publication No. WO 2009/047356, and International Patent Publication No. WO 2010/130830.

### DETAILED DESCRIPTION OF THE INVENTION

The invention provides an anti-sclerostin antibody comprising a CDR-H1 of SEQ ID NO:245, a CDR-H2 of SEQ ID NO:246, a CDR-H3 of SEQ ID NO:247, a CDR-L1 of SEQ ID NO:78, a CDR-L2 of SEQ ID NO:79 and a CDR-L3 of SEQ ID NO:80, for use in a method for increasing bone mineral density in a postmenopausal woman, the method comprising administering to a postmenopausal woman suffering from osteoporosis said anti-sclerostin antibody in an amount and for a treatment period effective to increase lumbar vertebrae bone mineral density (BMD) at least 9% from pretreatment baseline at twelve months following initial administration of the anti-sclerostin antibody; wherein the amount of anti-sclerostin antibody administered is about 140 mg or about 210 mg and the amount of the anti-sclerostin antibody is administered at an interval no more frequent than once a month. The amount and the time of administration are effective to increase lumbar vertebrae BMD at least about 9% from pretreatment baseline at twelve months following initial administration of the anti-sclerostin antibody.

In evaluating the risk, presence, or progression of osteoporosis (or other ailments associated with loss of BMD), a patient's BMD generally is compared to the peak density of a 30-year old healthy adult (i.e., a "young adult"), creating the so-called "T-score." Typically, BMD is measured "total body" (e.g., head, trunk, arms, and legs) or at the hip (e.g., total hip and/or femoral neck), spine (e.g., lumbar vertebrae), wrist, finger, shin bone and/or heel. A patient may have different T-scores corresponding to different measurement sites (e.g., a lumbar vertebrae T-score, a total hip T-score, and a femoral neck T-score, all of which may comprise different values). A patient's BMD also may be compared to an "age-matched" bone density (see, e.g., World Health Organization Scientific Group on the Prevention and Management of Osteoporosis, "Prevention and management of osteoporosis: report of a WHO scientific group." WHO Technical Report Series; 921, Geneva, Switzerland (2003)). The difference between a patient's BMD and that of a healthy, young adult is conventionally referred to in terms of the multiple of a "standard deviation," which typically equals about 10% to about 12% decrease in bone density. The World Health Organization proposed four diagnostic categories based on BMD T-scores. A BMD value within 1 standard deviation of the young adult reference mean (T-score ≥ -1) is "normal." Low bone mass (osteopenia) is indicated by a BMD value more than 1 standard deviation below the young adult mean, but less than 2.5 standard deviations (T-score < -1 and > -2.5). A T-score of 2.5 standard deviations or more than 2.5 standard deviations below the norm supports a diagnosis of osteoporosis (T-score ≤ -2.5). If a patient additionally suffers from one or more fragility fractures, the patient qualifies as having severe osteoporosis.

In various embodiments of the inventive method, the postmenopausal woman has a lumbar vertebrae T-score ≤ -1, such as a lumbar vertebrae T-score < -1 and > -2.5 (e.g., a lumbar vertebrae T-score of less than or equal to -2) or a lumbar vertebrae T-score ≤ -2.5. Alternatively (or in addition), the postmenopausal woman has a total hip T-score ≤ -1, such as a total hip T-score < -1 and > -2.5 (e.g., a total hip T-score of less than or equal to -2) or a total hip T-score ≤ -2.5.

The postmenopausal woman is suffering from osteoporosis. Alternatively or in addition, the postmenopausal woman is at increased or high risk for fracture, i.e., the subject has a history of osteoporotic fracture or multiple risk factors for fracture. In various embodiments of the invention, the anti-sclerostin antibody is administered to the postmenopausal woman in an amount and for a time effective to reduce the risk of vertebral and/or nonvertebral fractures. Alternatively or in addition, the postmenopausal woman optionally failed or is intolerant to other available osteoporosis therapy such as, but not limited to, RANK ligand (RANKL) inhibitors, such as anti-RANKL antibody (e.g., PROLIA®); bisphosphonates (e.g., zoledronic acid (RECLAST®), alendronate sodium (FOSAMAX®), or ibandronate sodium (BONIVA®)); or parathyroid hormone or analogs thereof (e.g., teriparatide, a recombinant human parathyroid hormone analog (1-34) (FORTEO®)). In various embodiments, the postmenopausal women has been treated with any of the listed osteoporosis therapies listed above prior to administration of the anti-sclerostin antibody. Patients pre-treated with bisphosphonates have been shown to exhibit a blunted response to teriparatide (FORTEO®). Pre-treatment with bisphosphonates will not result in a similar blunted response to anti-sclerostin antibody treatment. Thus, patients pre-treated with bisphosphonates will enjoy the full therapeutic potential of anti-sclerostin antibody treatment.

The anti-sclerostin antibody is administered to the postmenopausal woman in an amount and for a time effective to achieve an increase in lumbar vertebrae BMD at least 9% from pretreatment baseline at twelve months following the initial administration of the anti-sclerostin antibody. Lumbar spine is one of many skeletal sites for BMD measurement. Other suitable skeletal sites include hip (e.g., total hip or femoral neck), forearm, finger, wrist and heel. "Pretreatment baseline" refers to BMD measurement at the skeletal site prior to the initial administration of the anti-sclerostin antibody. Thus, BMD is measured at a skeletal site prior to treatment with anti-sclerostin antibody to obtain a baseline BMD measurement, which is compared to BMD measured at the same skeletal site at a timepoint after initial administration of the anti-sclerostin antibody, such as twelve months after the initial administration. In various aspects of the invention, the anti-sclerostin antibody is administered in an amount and for a time effective to increase lumbar vertebrae BMD (or BMD at another skeletal site) at least one standard deviation (e.g., 1.5 standard deviations, 2 standard deviations, or 2.5 standard deviations) from pretreatment baseline at twelve months following the initial administration of the anti-sclerostin antibody. In addition, the anti-sclerostin antibody may be administered in an amount and for a time effective to increase total hip BMD at least about 3% from pretreatment baseline at twelve months following initial administration of the anti-sclerostin antibody.

In humans, bone mineral density often is determined clinically using dual x-ray absorptiometry (DXA). Other techniques include quantitative computed tomography (QCT), ultrasonography, single-energy x-ray absorptiometry (SXA), magnetic resonance imaging, radiography, and radiographic absorptiometry. Except for ultrasonography, the American Medical Association notes that BMD techniques typically involve the use of x-rays and are based on the principle that attenuation of the radiation depends on thickness and composition of the tissues in the radiation path. Often, techniques involve the comparison of results to a normative database.

The amount of anti-sclerostin antibody in an administration to the postmenopausal woman as disclosed comprises at least about 70 mg of the anti-sclerostin antibody. For example, in various aspects, the amount of anti-sclerostin antibody administered is about 140 mg, or about 210 mg anti-sclerostin antibody. The amount of anti-sclerostin antibody administered in a method of the disclosure is no more than about 350 mg anti-sclerostin antibody, e.g., no more than about 280 mg anti-sclerostin antibody, no more than about 210 mg of anti-sclerostin antibody, no more than about 140 mg anti-sclerostin antibody, or no more than about 120 mg anti-sclerostin antibody (e.g., about 120 mg of antibody). Put another way, a single administration or dose of anti-sclerostin comprises, for example, no more than about 350 mg of the antibody (although the single dose may be administered via multiple contemporaneous injections (e.g., two injections of 105 mg of antibody to achieve a dose of 210 mg)). Thus, in various embodiments of the disclosure, the method comprises administering anti-sclerostin antibody in an amount of about 70 mg to about 350 mg, such as about 70 mg to about 280 mg, or about 120 mg to about 350 mg, or about 140 mg to about 350 mg, or about 210 mg to about 350 mg, or about 280 mg to about 350 mg. Optionally, a single dose of anti-sclerostin antibody comprises about 70 mg to about 210 mg of anti-sclerostin antibody, such as about 70 mg to about 120 mg (e.g., about 70 mg) anti-sclerostin antibody, or about 70 mg to about 140 mg of anti-sclerostin antibody, or about 120 mg to about 210 mg anti-sclerostin antibody, or about 120 mg to about 140 mg of anti-sclerostin antibody. A single dose of anti-sclerostin antibody comprises 140 mg to 210 mg (e.g., about 140 mg or about 210 mg) of anti-sclerostin antibody. An amount of anti-sclerostin antibody (i.e., dose of anti-sclerostin antibody) is administered at an interval no more frequent than once a month (or four weeks), optionally for a treatment period of at least about three months (or 12 weeks). The treatment period comprises no more than about 18 months (i.e., about three months to about 18 months). In other words, a waiting period of at least one month passes between anti-sclerostin antibody administrations to the postmenopausal woman, and the course of treatment optionally lasts at least about three months and no longer than about 18 months (e.g., about three months to about 18 months). In various aspects, the multiple doses of the anti-sclerostin antibody are administered over a treatment period of about three months to about 15 months, or about three months to about 12 months, or about three months to about nine months, or about three months to about six months, or about six months to about 18 months, or about six months to about 15 months, or about six months to about 12 months, or about six months to about 9 months, or about 9 months to about 18 months, or about 9 months to about 15 months, or about 9 months to about 12 months, or about 12 months to about 18 months, or about 12 months to about 15 months, or about 15 months to about 18 months (e.g., about three months, about six months, about nine months, about 12 months, about 15 months, or about 18 months). Optionally, the multiple doses of the anti-sclerostin antibody is administered over a treatment period of about 12 weeks to about 52 weeks, or about 12 weeks to about 36 weeks, or about 12 weeks to about 24 weeks, with doses administered once a week, once every two weeks, once every four weeks, once every six weeks, once every 8 weeks, or once every 12 weeks. In one aspect of the disclosure, the amount of anti-sclerostin antibody is administered at an interval no more frequent than once every three months (e.g., once every 12 weeks), optionally for a treatment period of about six months to about 18 months.

The dosing and timing regimen described herein (e.g., about 70 mg, about 140 mg, or about 210 mg, administered once monthly (QM) or every three months (Q3M) over a treatment period of, for example, about 12 months) has been shown to be effective in increasing bone mineral density in the lumbar vertebrae by at least about 5% (e.g., at least about 6%, at least about 7%, at least about 8%, at least about 9%, at least about 10%, or at least about 11%) at 12 months following initial administration of the anti-sclerostin antibody, and/or at least about 4% (e.g., at least about 5%, at least about 6%, at least about 7%, or at least about 8%) at 6 months following initial administration of the anti-sclerostin antibody. For example, when administered subcutaneously in an amount of about 210 mg once a month for three months, the anti-sclerostin antibody increases lumbar vertebrae bone mineral density by at least about 5%. The dosing and timing regimen of the inventive method has been shown to be effective in increasing total hip bone mineral density by at least about 1% (e.g., at least about 2%, at least about 3%, or at least about 4%) at 12 months following initial administration of the anti-sclerostin antibody. The dosing and timing regimen described herein has been shown to be effective in increasing femoral neck bone mineral density by at least about 0.5% (e.g., at least about 1%, at least about 1.5%, at least about 2%, at least about 2.5%, at least about 3%, at least about 3.5%, or at least about 4%) at 12 months following initial administration of the anti-sclerostin antibody.

A subject's physiological response to an anti-sclerostin antibody can be gauged by monitoring bone marker levels. Bone markers are products created during the bone remodeling process and are released by bone, osteoblasts, and/or osteoclasts. Fluctuations in bone resorption and/or bone formation "marker" levels imply changes in bone remodeling/modeling. The International Osteoporosis Foundation (IOF) recommends using bone markers to monitor bone density therapies (see, e.g., Delmas et al., Osteoporos Int., Suppl. 6:S2-17 (2000). Markers indicative of bone resorption (or osteoclast activity) include, for example, C-telopeptide (e.g., C-terminal telopeptide of type 1 collagen (CTX) or serum cross-linked C-telopeptide), N-telopeptide (N-terminal telopeptide of type 1 collagen (NTX)), deoxypyridinoline (DPD), pyridinoline, urinary hydroxyproline, galactosyl hydroxylysine, and tartrate-resistant acid phosphatase (e.g., serum tartrate-resistant acid phosphatase isoform 5b). Bone formation/mineralization markers include, but are not limited to, bone-specific alkaline phosphatase (BSAP), peptides released from N- and C-terminal extension of type I procollagen (P1NP, PICP), and osteocalcin (OC). Several kits are commercially-available to detect and quantify markers in clinical samples, such as urine and blood.

In various aspects of the invention, administration of the anti-sclerostin antibody results in an increase in bone formation marker levels in, for example, the serum and/or urine of a treated subject. Generally, the amount of bone formation markers peak within one to two months of the initial administration of the anti-sclerostin antibody and fade over subsequent (e.g., two to six) months, regardless of whether subsequent doses of anti-sclerostin antibody are administered. In Example 2, for example, P1NP, BSAP, and OC levels fell to approximately baseline (amount of marker detected prior to the initial administration of the anti-sclerostin antibody) at about two to six months after the initial administration of the anti-sclerostin antibody, despite repeated administrations of the antibody over the same time period. Simultaneously, levels of a bone resorption marker (e.g., CTX) decrease following administration of the anti-sclerostin antibody, indicating an uncoupling of bone formation and bone resorption during treatment. In Example 2, for instance, serum CTX levels dropped below baseline as soon as one week following the initial anti-sclerostin antibody administration, and remained below baseline throughout a twelve month treatment period. Also surprisingly, BMD continues to elevate after bone formation and resorption markers approach baseline levels.

In one aspect, the disclosure provides a method of treating osteoporosis. A postmenopausal woman with osteoporosis may be administered an anti-sclerostin antibody in an amount of about 210 mg at an interval of once a month. In various aspects, the postmenopausal woman is administered a once monthly dose of about 140 mg of anti-sclerostin antibody, or a once monthly dose of about 210 mg of anti-sclerostin antibody. Multiple administrations (i.e., doses) of the anti-sclerostin antibody are administered to the postmenopausal woman over a treatment period of, e.g., about three months to about 18 months, such as a treatment period of about three months to about 15 months, or about three months to about 12 months, or about three months to about nine months, or about three months to about six months, or about six months to about 18 months, or about six months to about 15 months, or about six months to about 12 months, or about six months to about 9 months, or about 9 months to about 18 months, or about 9 months to about 15 months, or about 9 months to about 12 months, or about 12 months to about 18 months, or about 12 months to about 15 months, or about 15 months to about 18 months (e.g., about three months, about six months, about nine months, about 12 months, about 15 months, or about 18 months). Optionally, multiple doses of an anti-sclerostin antibody are administered over a treatment period of about 12 weeks to about 52 weeks, or about 12 weeks to about 36 weeks, or about 12 weeks to about 24 weeks, with doses administered once a week, once every two weeks, once every four weeks, once every six weeks, once every 8 weeks, or once every 12 weeks. In various embodiments, the anti-sclerostin antibody is administered over a treatment period consisting of 12 months or 18 months.

Alternatively, the method of the disclosure comprises administering to a postmenopausal woman with osteoporosis an anti-sclerostin antibody in an amount of about 120 mg to about 210 mg or in an amount of about 140 mg to about 210 mg, at an interval of every three months. In this regard, the postmenopausal woman is optionally administered a dose of about 140 mg of anti-sclerostin antibody or a dose of about 210 mg of anti-sclerostin antibody every three months (e.g., about every 90 days). Multiple administrations (i.e., doses) of the anti-sclerostin antibody are administered to the postmenopausal woman over a treatment period about six months to about 18 months in length, such as a treatment period of about six months to about 15 months, or about six months to about 12 months, or about six months to about 9 months, or about 9 months to about 18 months, or about 9 months to about 15 months, or about 9 months to about 12 months, or about 12 months to about 18 months, or about 12 months to about 15 months, or about 15 months to about 18 months (e.g., about three months, about six months, about nine months, about 12 months, about 15 months, or about 18 months). In various embodiments, the anti-sclerostin antibody is administered over a treatment period consisting of 12 months or 18 months.

By "treating osteoporosis" is meant the amelioration, in whole or in part, of osteoporosis, or protection, in whole or in part, against further progression of osteoporosis. Complete reversal of the disease is not required in the context of the invention; any therapeutic benefit, including improvement in BMD and/or reduction in risk of fracture, is contemplated.

The term "antibody" refers to an intact antibody, or a binding fragment thereof. An antibody may comprise a complete antibody (immunoglobulin) molecule (including polyclonal, monoclonal, chimeric, humanized, and/or human versions having full length heavy and/or light chains (e.g., an IgG antibody)), or comprise an antigen binding fragment thereof. Antibody fragments include F(ab')₂, Fab, Fab', Fv, Fc, and Fd fragments, and can be incorporated into single domain antibodies (e.g., nanobodies), single-chain antibodies, maxibodies, minibodies, intrabodies, diabodies, triabodies, tetrabodies, v-NAR and bis-scFv (see, e.g., Hollinger and Hudson, Nature Biotechnology, 23(9):1126-1136 (2005)). Antibody polypeptides, including fibronectin polypeptide monobodies, also are disclosed in U.S. Patent No. 6,703,199. Other antibody polypeptides are disclosed in U.S. Patent Publication No. 20050238646. U.S. Patent Nos. 6,395,511 and 6,803,453, and U.S. Patent Publication Nos. 20040009535 and 20050106683 refer to anti-sclerostin antibodies generally. The amino acid sequence of human sclerostin is set forth in SEQ ID NO: 1 of the Sequence Listing and is provided as SEQ ID NO: 1 of U.S. Patent Publication No. 20070110747. Sclerostin also is described in Brunkow et al., Am. J. Hum. Genet., 68:577-589 (2001); and Balemans et al., Hum. Mol. Genet., 10:537-543 (2001). Additional information regarding materials and methods for generating anti-sclerostin antibodies can be found in U.S. Patent Publication No. 20040158045.

An antibody fragment may be any synthetic or genetically engineered protein. For example, antibody fragments include isolated fragments consisting of the light chain or heavy chain variable region, "Fv" fragments consisting of the variable regions of the heavy and light chains, and recombinant single chain polypeptide molecules in which light and heavy variable regions are connected by a peptide linker (scFv proteins).

Another form of an antibody fragment is a peptide comprising one or more complementarity determining regions (CDRs) of an antibody. CDRs (also termed "minimal recognition units" or "hypervariable region") can be obtained by constructing polynucleotides that encode the CDR of interest. Such polynucleotides are prepared, for example, by using the polymerase chain reaction to synthesize the variable region using mRNA of antibody-producing cells as a template (see, for example, Larrick et al., Methods: A Companion to Methods in Enzymology, 2:106 (1991); Courtenay-Luck, "Genetic Manipulation of Monoclonal Antibodies," in Monoclonal Antibodies Production, Engineering and Clinical Application, Ritter et al. (eds.), page 166, Cambridge University Press (1995); and Ward et al., "Genetic Manipulation and Expression of Antibodies," in Monoclonal Antibodies: Principles and Applications, Birch et al., (eds.), page 137, Wiley-Liss, Inc. (1995)).

Anti-sclerostin antibodies may bind to sclerostin of SEQ ID NO: 1, or a naturally occurring variant thereof, with an affinity (Kd) of less than or equal to 1 x 10⁻⁷ M, less than or equal to 1 x 10⁻⁸ M, less than or equal to 1 x 10⁻⁹ M, less than or equal to 1 x 10⁻¹⁰ M, less than or equal to 1 x 10⁻¹¹ M, or less than or equal to 1 x 10⁻¹² M. Affinity is determined using a variety of techniques, an example of which is an affinity ELISA assay. In various embodiments, affinity is determined by a surface plasmon resonance assay (e.g., a BIAcore™ assay). In various embodiments, affinity is determined by a kinetic method. In various embodiments, affinity is determined by an equilibrium/solution method. U.S. Patent Publication No. 20070110747 contains additional description of affinity assays suitable for determining the affinity (Kd) of an antibody for sclerostin.

Anti-sclerostin antibodies for use in the inventive method preferably modulate sclerostin function in the cell-based assay described in U.S. Patent Publication No. 20070110747 and/or the *in vivo* assay described in U.S. Patent Publication No. 20070110747 and/or anti-sclerostin antibody for use in the disclosure method bind to one or more of the epitopes described in U.S. Patent Publication No. 20070110747 and/or cross-block the binding of one of the antibodies described in U.S. Patent Publication No. 20070110747 and/or are cross-blocked from binding sclerostin by one of the antibodies described in U.S. Patent Publication No. 20070110747.

In various embodiments, the anti-sclerostin antibody binds to a sclerostin polypeptide comprising the amino acid sequence set forth in SEQ ID NO: 1 and binds the sequence of SEQ ID NO: 6 (C4GPARLLPNAIGRGKWWRPSGPDFRC5; corresponding to amino acids 86-111 of SEQ ID NO: 1). Alternatively or in addition, the anti-sclerostin antibody of the disclosure binds to a sclerostin polypeptide comprising the amino acid sequence set forth in SEQ ID NO: 1 and binds the sequence of at least one of SEQ ID NO: 2 (DVSEYSC1RELHFTR; corresponding to amino acids 51-64 of SEQ ID NO: 1), SEQ ID NO: 3 (SAKPVTELVC3SGQC4GPAR; corresponding to amino acids 73-90 of SEQ ID NO: 1), SEQ ID NO: 4 (WWRPSGPDFRC5IPDRYR; corresponding to amino acids 101-117 of SEQ ID NO: 1), SEQ ID NO: 5 (LVASC7KC8KRLTR; corresponding to amino acids 138-149 of SEQ ID NO: 1), SEQ ID NO: 70 (SAKPVTELVC3SGQC4; corresponding to amino acids 73-86 of SEQ ID NO: 1), SEQ ID NO: 71 (LVASC7KC8; corresponding to amino acids 138-144 of SEQ ID NO: 1), SEQ ID NO: 72 (C1RELHFTR; corresponding to amino acids 57-64 of SEQ ID NO: 1), or SEQ ID NO: 73 (C5IPDRYR; corresponding to amino acids 111-117 of SEQ ID NO: 1) within SEQ ID NO: 1. For example, in one aspect, the anti-sclerostin antibody binds a subregion of sclerostin of SEQ ID NO: 1 comprising SEQ ID NOs: 2-5 (and/or SEQ ID NOs: 70-73), optionally in its native three-dimensional conformation. Optionally, the anti-sclerostin antibody of the disclosure binds a peptide consisting of one or more of SEQ ID NO: 2, SEQ ID NO: 3, SEQ ID NO: 4, SEQ ID NO: 5, SEQ ID NO: 6, SEQ ID NO: 70, SEQ ID NO: 71, SEQ ID NO: 72, or SEQ ID NO: 73 (e.g., a peptide consisting of SEQ ID NO: 2, SEQ ID NO: 3, SEQ ID NO: 4, and SEQ ID NO: 5 or a peptide consisting of SEQ ID NO: 70, SEQ ID NO: 71, SEQ ID NO: 72, and SEQ ID NO: 73).

In various aspects, the anti-sclerostin antibody is capable of neutralizing human sclerostin in a MC3T3 cell-based mineralization assay when there is less than a 6-fold excess of moles of sclerostin binding sites per well as compared to the number of moles of sclerostin per well. Mineralization by osteoblast-lineage cells in culture, either primary cells or cell lines, is used as an *in vitro* model of bone formation. An exemplary cell-based mineralization assay is described in U.S. Patent Publication No. 20070110747 at, e.g., Example 8. MC3T3-E1 cells (Sudo et al., J. Cell Biol., 96:191-198 (1983)) and subclones of the original cell line can form mineral in culture upon growth in the presence of differentiating agents. Such subclones include MC3T3-E1-BF (Smith et al., J. Biol. Chem., 275:19992-20001 (2000)). For both the MC3T3-E1-BF subclone as well as the original MC3T3-E1 cells, sclerostin can inhibit one or more of the sequence of events leading up to and including mineral deposition (i.e., sclerostin inhibits mineralization). Anti-sclerostin antibodies that are able to neutralize sclerostin's inhibitory activity allow for mineralization of the culture in the presence of sclerostin such that there is a statistically significant increase in, e.g., deposition of calcium phosphate (measured as calcium) as compared to the amount of calcium measured in the sclerostin-only (i.e., no antibody) treatment group.

When running the assay with the goal of determining whether a particular anti-sclerostin antibody can neutralize sclerostin, the amount of sclerostin used in the assay desirably is the minimum amount of sclerostin that causes at least a 70%, statistically significant, reduction in deposition of calcium phosphate (measured as calcium) in the sclerostin-only group, as compared to the amount of calcium measured in the no sclerostin group. An anti-sclerostin neutralizing antibody is defined as one that causes a statistically significant increase in deposition of calcium phosphate (measured as calcium) as compared to the amount of calcium measured in the sclerostin-only (i.e., no antibody) treatment group. To determine whether an anti-sclerostin antibody is neutralizing or not, the amount of anti-sclerostin antibody used in the assay needs to be such that there is an excess of moles of sclerostin binding sites per well as compared to the number of moles of sclerostin per well. Depending on the potency of the antibody, the fold excess that may be required can be 24, 18, 12, 6, 3, or 1.5, and one of skill is familiar with the routine practice of testing more than one concentration of binding agent (antibody). For example, a very potent anti-sclerostin neutralizing antibody will neutralize sclerostin when there is less than a 6-fold excess of moles of sclerostin binding sites per well as compared to the number of moles of sclerostin per well. A less potent anti-sclerostin neutralizing antibody will neutralize sclerostin only at a 12, 18, or 24 fold excess.

The anti-sclerostin antibody optionally has an IC₅₀ of 100 nM or less, or 75 nM or less, or 50 nM or less, or 25 nM or less for neutralizing human sclerostin in a cell-based assay, such as a bone specific alkaline phosphatase assay, e.g., the bone specific alkaline phosphatase assay described in International Patent Publication No. WO 2008/115732 and U.S. Patent No. 7,744,874. The bone specific alkaline phosphatase assay is predicated on the ability of sclerostin to decrease BMP-4 and Wnt3a-stimulated alkaline phosphatase levels in the multipotential murine cell line, C2C12. According to WO 2008/115732, a neutralizing anti-sclerostin antibody mediates a dose-dependent increase of alkaline phosphatase activity in this assay. Exemplary protocols of the cell-based assays are provided in Example 1.

Alternatively or in addition, the anti-sclerostin antibody has an IC₅₀ of 100 nM or less (e.g., 75 nM or less, or 50 nM or less) for neutralizing human sclerostin in a cell-based Wnt signalling assay in HEK293 cell lines, such as the Wnt assay involving Wnt1-mediated induction of STF reporter gene described in e.g., International Patent Publication No. WO 2009/047356. Alternatively or in addition, the anti-sclerostin antibody has an IC₅₀ of 500 nM or less (e.g., 250 nM or less, 150 nM or less, 100 nM or less, or 50 nM or less) for neutralizing human sclerostin in a BMP2-induced mineralization assay in MC3T3 cells, such as the mineralization assay described in e.g., International Patent Publication No. WO 2009/047356. An exemplary protocol is provided in Example 1.

Examples of anti-sclerostin antibodies suitable for use in the context of the disclosure are described in U.S. Patent Publication Nos. 20070110747 and 20070072797. In one embodiment of the invention, the anti-sclerostin antibody cross-blocks the binding of at least one of antibodies Ab-A, Ab-B, Ab-1, Ab-4, Ab-5, Ab-6, Ab-7, Ab-8, Ab-9, Ab-10, Ab-12, Ab-19, Ab-20, Ab-23, or Ab-24 (all of which are described in U.S. Patent Publication No. 20070110747, and the amino acid sequences of which are provided in Figure 1) to sclerostin. Alternatively or in addition, the anti-sclerostin antibody is cross-blocked from binding to sclerostin by at least one of antibodies Ab-A, Ab-B, Ab-1, Ab-4, Ab-5, Ab-6, Ab-7, Ab-8, Ab-9, Ab-10, Ab-12, Ab-19, Ab-20, Ab-23, or Ab-24 (all of which are described in U.S. Patent Publication No. 20070110747). In one embodiment of the disclosure, the anti-sclerostin antibody cross-blocks the binding of a reference antibody to sclerostin of SEQ ID NO: 1 or is cross-blocked from binding to sclerostin of SEQ ID NO: 1 by the reference antibody, wherein the reference antibody comprises (a) light chains comprising the amino acid sequence set forth in SEQ ID NO: 205 and heavy chains comprising the amino acid sequence set forth in SEQ ID NO: 209; (b) light chains comprising the amino acid sequence set forth in SEQ ID NO: 15 and heavy chains comprising the amino acid sequence set forth in SEQ ID NO: 19; or (c) light chains comprising the amino acid sequence set forth in SEQ ID NO: 7 and heavy chains comprising the amino acid sequence set forth in SEQ ID NO: 11.

The terms "cross-block," "cross-blocked," and "cross-blocking" are used interchangeably herein to mean the ability of an antibody to interfere with the binding of other antibodies to sclerostin. The extent to which an antibody is able to interfere with the binding of another to sclerostin, and therefore whether it can be said to cross-block, can be determined using competition binding assays. In some aspects of the invention, a cross-blocking antibody or fragment thereof reduces sclerostin binding of a reference antibody between about 40% and about 100%, such as about 60% and about 100%, specifically between 70% and 100%, and more specifically between 80% and 100%. A particularly suitable quantitative assay for detecting cross-blocking uses a Biacore machine which measures the extent of interactions using surface plasmon resonance technology. In this regard, the anti-sclerostin antibody optionally binds to sclerostin in a surface plasmon resonance assay such that during the assay in the presence of a reference antibody, the recorded binding is between 80% and 4% (e.g., between 75% and 4% or between 70% and 4%) of the maximum theoretical binding (i.e., the sum of the binding of each antibody when passed over the sclerostin surface alone). Another suitable quantitative cross-blocking assay uses an ELISA-based approach to measure competition between antibodies in terms of their binding to sclerostin. In this regard, the anti-sclerostin antibody in solution in an ELISA assay causes a reduction of between 60% and 100% (e.g., between 70% and 100% or between 80% and 100%) in the amount of sclerostin bound by an immobilized reference antibody and/or the reference antibody in solution in an ELISA assay causes a reduction of between 60% and 100% (e.g., between 70% and 100% or between 80% and 100%) in the amount of sclerostin bound by the anti-sclerostin antibody, compared to the amount of sclerostin bound in the absence of the anti-sclerostin antibody or reference antibody in solution. Assays for detecting cross-blocking of anti-sclerostin antibodies and a reference antibody are further described in U.S. Patent Publication No. 20070110747.

Examples of suitable anti-sclerostin antibodies and fragments thereof of the disclosure include antibodies and antibody fragments having one or more of CDR-H1, CDR-H2, CDR-H3, CDR-L1, CDR-L2 and CDR-L3 specifically disclosed in U.S. Patent Publication No. 20070110747. At least one of the regions of CDR-H1, CDR-H2, CDR-H3, CDR-L1, CDR-L2, and CDR-L3 may have at least one amino acid substitution (e.g., a conservative amino acid substitution), provided that the antibody retains the binding specificity of the non-substituted CDR. Preferably, the anti-sclerostin antibody is Ab-4 or Ab-5 of U.S. Patent Publication No. 20070110747.

In addition, the anti-sclerostin antibody of the disclosure can comprise at least one CDR sequence having at least 75% identity (e.g., 100% identity) to a CDR selected from SEQ ID NOs: 39, 40, 41, 42, 43, 44, 45, 46, 47, 48, 49, 50, 51, 52, 53, 54, 55, 56, 57, 58, 59, 60, 61, 62, 78, 79, 80, 81, 99, 100, 101, 102, 103, 104, 105, 106, 107, 108, 109, 110, 111, 112, 113, 114, 115, 116, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 351, 352, 353, 358, 359, and 360 provided in the Sequence Listing and disclosed in U.S. Patent Publication No. 20070110747. Preferably, the anti-sclerostin antibody of the disclosure comprises at least one CDR sequence having at least 75% identity to a CDR selected from SEQ ID NOs: 245, 246, 247, 78, 79, 80, 269, 270, 271, 239, 240, and 241, all of which is provided in the Sequence Listing and described in U.S. Patent Publication No. 20070110747. As described in U.S. Patent Publication No. 20070110747, the anti-sclerostin antibody of the disclosure can comprise: a) CDR sequences of SEQ ID NOs: 54, 55, and 56 and CDR sequences of SEQ ID NOs: 51, 52, and 53; b) CDR sequences of SEQ ID NOs: 60, 61, and 62 and CDR sequences of SEQ ID NOs: 57, 58, and 59; c) CDR sequences of SEQ ID NOs: 48, 49, and 50 and CDR sequences of SEQ ID NOs: 45, 46, and 47; d) CDR sequences of SEQ ID NOs: 42, 43, and 44 and CDR sequences of SEQ ID NOs: 39, 40, and 41; e) CDR sequences of SEQ ID NOs: 275, 276, and 277 and CDR sequences of SEQ ID NOs: 287, 288, and 289; f) CDR sequences of SEQ ID NOs: 278, 279, and 280 and CDR sequences of SEQ ID NOs: 290, 291, and 292; g) CDR sequences of SEQ ID NOs: 78, 79, and 80 and CDR sequences of SEQ ID NOs: 245, 246, and 247; h) CDR sequences of SEQ ID NOs: 81, 99, and 100 and CDR sequences of SEQ ID NOs :248, 249, and 250; i) CDR sequences of SEQ ID NOs: 101, 102, and 103 and CDR sequences of SEQ ID NOs: 251, 252, and 253; j) CDR sequences of SEQ ID NOs: 104, 105, and 106 and CDR sequences of SEQ ID NOs: 254, 255, and 256; k) CDR sequences of SEQ ID NOs: 107, 108, and 109 and CDR sequences of SEQ ID NOs: 257, 258, and 259; l) CDR sequences of SEQ ID NOs: 110, 111, and 112 and CDR sequences of SEQ ID NOs: 260, 261, and 262; m) CDR sequences of SEQ ID NOs: 281, 282, and 283 and CDR sequences of SEQ ID NOs: 293, 294, and 295; n) CDR sequences of SEQ ID NOs: 113, 114, and 115 and CDR sequences of SEQ ID NOs: 263, 264, and 265; o) CDR sequences of SEQ ID NOs: 284, 285, and 286 and CDR sequences of SEQ ID NOs: 296, 297, and 298; p) CDR sequences of SEQ ID NOs: 116, 237, and 238 and CDR sequences of SEQ ID NOs: 266, 267, and 268; q) CDR sequences of SEQ ID NOs: 239, 240, and 241 and CDR sequences of SEQ ID NOs: 269, 270, and 271; r) CDR sequences of SEQ ID NOs: 242, 243, and 244 and CDR sequences of SEQ ID NOs: 272, 273, and 274; or s) CDR sequences of SEQ ID NOs: 351, 352, and 353 and CDR sequences of SEQ ID NOs: 358, 359, and 360.

The anti-sclerostin antibody comprises CDR sequences having (100% identity) to CDRS of CDR-H1, CDR-H2, CDR-H3, CDR-L1, CDR-L2, and CDR-L3 wherein CDR-H1 has the sequence given in SEQ ID NO: 245, CDR-H2 has the sequence given in SEQ ID NO: 246, CDR-H3 has the sequence given in SEQ ID NO: 247, CDR-L1 has the sequence given in SEQ ID NO: 78, CDR-L2 has the sequence given in SEQ ID NO: 79 and CDR-L3 has the sequence given in SEQ ID NO: 80, all of which is provided in the Sequence Listing and described in U.S. Patent Publication No. 20070110747. The anti-sclerostin antibody, in various aspects of the disclosure, comprises two of the CDRs or three of the CDRs or six of the CDRs. Optionally, the anti-sclerostin antibody comprises heavy chains comprising SEQ ID NO: 378 and light chains comprising SEQ ID NO 376.

The anti-sclerostin antibody of the disclosure also can comprise at least one CDR sequence having at least 75% identity (e.g., 100% identical) to a CDR selected from CDR-H1, CDR-H2, CDR-H3, CDR-L1, CDR-L2, and CDR-L3 wherein CDR-H1 has the sequence given in SEQ ID NO: 269, CDR-H2 has the sequence given in SEQ ID NO: 270, CDR-H3 has the sequence given in SEQ ID NO: 271, CDR-L1 has the sequence given in SEQ ID NO: 239, CDR-L2 has the sequence given in SEQ ID NO: 240 and CDR-L3 has the sequence given in SEQ ID NO 241, all of which is provided in the Sequence Listing and described in U.S. Patent Publication No. 20070110747. The anti-sclerostin antibody, in various aspects of the disclosure, comprises two of the CDRs or three of the CDRs or six of the CDRs.

Alternatively, the anti-sclerostin antibody can have a heavy chain comprising CDR's H1, H2, and H3 and comprising a polypeptide having the sequence provided in SEQ ID NO: 137 or a variant thereof in which said CDR's are 100% identical to SEQ ID NO: 245, 246, and 247, respectively, and a light chain comprising CDR's LI, L2 and L3 and comprising a polypeptide having the sequence provided in SEQ ID NO: 133 or a variant thereof in which said CDR's are 100% identical to SEQ ID NO: 78, 79, and 80, respectively (as described in U.S. Patent Publication No. 20070110747).

The anti-sclerostin antibody may have a heavy chain comprising CDR's H1, H2, and H3 and comprising a polypeptide having the sequence provided in SEQ ID NO: 145 or 392 or a variant thereof in which said CDR's are 100% identical to SEQ ID NO: 245, 246, and 247, respectively, and a light chain comprising CDR's LI, L2, and L3 and comprising a polypeptide having the sequence provided in SEQ ID NO: 141 or a variant thereof in which said CDR's are 100% identical to SEQ ID NO: 78, 79, and 80, respectively (as described in U.S. Patent Publication No. 20070110747).

The anti-sclerostin antibody of the disclosure may have a heavy chain comprising CDR's H1, H2, and H3 and comprising a polypeptide having the sequence provided in SEQ ID NO: 335, 331, 345, or 396 or a variant of any of the foregoing in which said CDR's are at least 75% (e.g., 100% identical) identical to SEQ ID NO: 269, 270, and 271, respectively, and a light chain comprising CDR's LI, L2, and L3 and comprising a polypeptide having the sequence provided in SEQ ID NO: 334 or 341 or a variant of any of the foregoing in which said CDR's are at least 75% identical (e.g., 100% identical) to SEQ ID NO: 239, 240, and 241, respectively (as described in U.S. Patent Publication No. 20070110747). All combinations of the heavy and light chain sequences are contemplated by the disclosure (e.g., heavy chains comprising SEQ ID NO: 335 and light chains comprising SEQ ID NO: 334; heavy chains comprising SEQ ID NO: 331 and light chains comprising SEQ ID NO: 334 or 341; and heavy chains comprising SEQ ID NO: 345 or 396 and light chains comprising SEQ ID NO: 341). Sequence identity of variants can comprise, e.g., at least about 75%, at least about 85%, at least about 90%, at least about 95% or 100% identity to a reference sequence.

The anti-sclerostin antibody may have a heavy chain comprising a polypeptide having the sequence provided in SEQ ID NO: 145 or 392, and a light chain comprising a polypeptide having the sequence provided in SEQ ID NO: 141 (as described in U.S. Patent Publication No. 20070110747).

Examples of anti-sclerostin antibodies also include, but are not limited to, the anti-sclerostin antibodies disclosed in International Patent Publication Nos. WO 2008/092894, WO 2008/115732, WO 2009/056634, WO 2009/047356, WO 2010/100200, WO 2010/100179, WO 2010/115932, and WO 2010/130830, such as an anti-sclerostin antibody comprising CDRs of SEQ ID NOs: 20-25 of International Patent Publication No. WO 2008/115732 (SEQ ID NOs: 416-421 herein), an anti-sclerostin antibody comprising CDRs of SEQ ID NOs: 26-31 of International Patent Publication No. WO 2008/115732 (SEQ ID NOs: 422-427 herein), an anti-sclerostin antibody comprising CDRs of SEQ ID NOs: 32-37 of International Patent Publication No. WO 2008/115732 (SEQ ID NOs: 428-433 herein), an anti-sclerostin antibody comprising CDRs of SEQ ID NOs: 4, 15, 26, 37, 48, and 59 of International Patent Publication No. WO 2009/047356 (SEQ ID NOs: 443, 454, 465, 476, 487, and 498 herein), or an anti-sclerostin antibody comprising the amino acid sequence of at least one of SEQ ID NOs: 135-143, 153-161, or 171-179 of International Patent Publication No. WO 2010/130830 (SEQ ID NOs: 745-753, 763-771, or 781-789 herein).

The invention also includes administering to a subject an anti-sclerostin antibody in combination with one or more additionally suitable agent(s), each being administered according to a regimen suitable for that active agent. Administration strategies include concurrent administration (i.e., substantially simultaneous administration) and non-concurrent administration (i.e., administration at different times, in any order, whether overlapping or not) of the anti-sclerostin antibody and one or more additionally suitable agents(s). It will be appreciated that different components are optionally administered in the same or in separate compositions, and by the same or different routes of administration.

Optionally, the additional agent(s) employ a mechanism of action that differs from the anti-sclerostin antibody, and provide a benefit to the subject. Suitable additional agent(s) include, but are not limited to calcium; vitamins (e.g., Vitamin D); anti-resorptives, such as RANKL inhibitors (e.g., anti-RANKL antibodies such as PROLIA®), bisphosphonates (e.g., alendronate sodium (FOSAMAX®), risedronate (ACTONEL®), ibandronate sodium (BONIVA®), or zoledronic acid (RECLAST®)), calcitonin, strontium compounds (e.g., strontium ranelate), selective estrogen receptor modulators (SERMs) (e.g., raloxifene), cathepsin K antagonists (e.g., odanacatib), and estrogen derivatives (e.g., conjugated equine estrogen)); anabolic agents, such as parathyroid hormone or analogs thereof (e.g., teriparatide (FORTEO®); antibiotics; hormone therapy; and anti-inflammatory agents (e.g., Non-Steroidal Anti-Inflammatory Drugs (NSAIDs) or steroidal anti-inflammatory substances). The amount of additional agent will vary depending on the subject and the presence or absence of additional therapeutic needs. For example, in various embodiments, the subject is administered at least about 1000 mg calcium and at least about 400 IU Vitamin D daily during the treatment period. Subjects having a Vitamin D level below 40 ng/mL are optionally administered an initial dose of 50,000 IU of Vitamin D. In various embodiments, the one or more additional agent(s) are administered following the treatment period as a maintenance therapy.

Various routes of administering an antibody to a subject are known in the art and discussed in, e.g., U.S. Patent Publication No. 20070110747. For example, in various embodiments, it is desirable to deliver a pharmaceutical composition comprising the anti-sclerostin antibody subcutaneously, parenterally, intravenously, intramuscularly, or even intraperitoneally. Such approaches are well known to the skilled artisan, some of which are further described, for example, in U.S. Patent Nos. 5,543,158; 5,641,515; and 5,399,363. Optionally, the anti-sclerostin antibody is administered to the postmenopausal women subcutaneously. Illustrative physiologically-acceptable (e.g., pharmaceutical) forms suitable for use include sterile aqueous solutions or dispersions and sterile powders for the extemporaneous preparation of sterile injectable solutions or dispersions (for example, see U.S. Patent No. 5,466,468). The form must be sterile and is desirably fluid to the extent that easy syringability exists (i.e., is not excessively viscous so as to prevent passage through a syringe). Any volume of carrier is appropriate for administering the anti-sclerostin antibody; in various embodiments, the anti-sclerostin antibody is administered subcutaneously in a volume of two milliliters or less (e.g., 1.5 mL or less, 1 mL or less, 0.88 mL or less, or 0.5 mL or less). Multiple injections may be employed to deliver a desired dose (e.g., a desired dose of 210 mg of anti-sclerostin antibody is administered via two subcutaneous injections of 105 mg anti-sclerostin antibody dissolved in 0.88 mL of carrier). A pharmaceutical composition comprising the anti-sclerostin antibody may be placed within containers (e.g., vials or syringes), along with packaging material that provides instructions regarding the use of such pharmaceutical compositions. Generally, such instructions will include a tangible expression describing the reagent concentration, as well as within certain embodiments, relative amounts of excipient ingredients or diluents (e.g., water, saline or PBS) that may be necessary to reconstitute the pharmaceutical composition.

The invention is further described in the following examples. The example serves only to illustrate the invention and are not intended to limit the scope of the invention in any way.

### EXAMPLES

### Example 1

This example describes various cell-based neutralization assays useful for characterizing the neutralization activity of an anti-sclerostin antibody.

*MC3T3 cell-based mineralization assay* - Ascorbic acid and B-glycerophosphate are used to induce MC3T3-E1-BF cell differentiation leading to mineral deposition. An exemplary screening protocol, in 96-well format, involves plating cells on day 1, followed by seven media changes over a 12-day period with most of the mineral deposition taking place in the final eighteen hours. The specific timing, and extent, of mineral deposition may vary depending, in part, on the particular serum lot number being used. Control experiments will allow such variables to be accounted for, as is well known in the art of cell culture experimentation generally. For statistical analysis (using MS Excel and JMP) a 1-way-ANOVA followed by Dunnett's comparison may be used to determine differences between groups. Group means for each data set are considered significantly different when the P value is less than 0.05 (P < 0.05).

Cell culture for expansion of MC3T3-E1-BF cells is performed as follows. Cell culture is performed at 37°C and 5% CO₂. A cell bank can be generated for the purposes of screening for sclerostin neutralizing antibodies. One vial of frozen MC3T3-E1-BF cells are thawed by agitation in a 37°C water bath. The thawed cells are put into 10 mls of Expansion Medium (Alpha-MEM/10%FBS/PenStrepGlu) in a 50 ml tube and gently spun down for 5 minutes. The cells are then resuspended in 4 mls of Alpha-MEM/10%FBS/PenStrepGlu. After determining the number of cells using trypan blue and hemacytometer, 1 x 10⁶ cells are plated in 50 mls Alpha-MEM/10%FBS/PenStrepGlu media in one T175 flask.

When this passage is confluent (at approximately 7 days), the cells are trypsinized with trypsin/EDTA (0.05% Trypsin; 0.53 mM EDTA), gently spun down for 5 minutes and then resuspended in 5 mls Alpha-MEM/10%FBS/PenStrepGlu. After determining the number of cells using trypan blue and hemacytometer, cells are plated at 1 x 10⁶ cells in 50 mls Alpha-MEM/10%FBS/PenStrepGlu media per one T175 flask. The number of T175 flasks used for plating at this point depends upon the total cell number available and the desired number of flasks that are to be taken forward to the next passage.

When this passage is confluent (about 3-4 days), the cells are trypsinized with trypsin/EDTA (0.05% Trypsin; 0.53 mM EDTA), gently spun down for 5 minutes and then resuspended in 5 mls Alpha-MEM/10%FBS/PenStrepGlu. After determining the number of cells using trypan blue and hemacytometer, cells are plated at 1 x 10⁶ cells in 50 mls Alpha-MEM/10%FBS/PenStrepGlu media per one T175 flask. The number of T175 flasks used for plating at this point depends upon the total cell number available and the desired number of flasks that were to be taken forward to the next passage.

When this passage is confluent (about 3-4 days), the cells are trypsinized with trypsin/EDTA (0.05% Trypsin; 0.53 mM EDTA), gently spun down for 5 minutes and then resuspended in 5 mls Alpha-MEM/10%FBS/PenStrepGlu. After determining the number of cells using trypan blue and hemacytometer, cells are plated at 1 x 10⁶ cells in 50 mls Alpha-MEM/10%FBS/PenStrepGlu media per one T175 flask. The number of T175 flasks used for plating at this point depends upon the total cell number available and the desired number of flasks that were to be taken forward to the next passage. Extra cells are frozen down at 1-2 x 10⁶ live cells/ml in 90%FBS/10%DMSO.

When this passage is confluent (about 3-4 days), the cells are trypsinized with trypsin/EDTA (0.05% Trypsin; 0.53 mM EDTA), gently spun down for 5 minutes and then resuspended in 5 mls Alpha-MEM/10%FBS/PenStrepGlu. After determining the number of cells using trypan blue and hemacytometer, the cells are frozen down at 1-2 x 10⁶ live cells/ml in 90%FBS/10%DMSO. This "final passage" of frozen cells is the passage used for the screening assay.

Cell culture for mineralizing MC3T3-E1-BF cells is performed as follows. Cell culture is performed at 37°C and 5% CO₂. It is desirable to minimize temperature and % CO₂ fluctuations during the mineralization cell culture procedure. An appropriate number of "final passage" vials prepared as described above are thawed by agitation in a 37°C water bath. The thawed cells are put into 10 mls of Expansion Medium (Alpha-MEM/10%FBS/PenStrepGlu) in a 50 ml tube and gently spun down for 5 minutes. The cells are then resuspended in 4 mls of Alpha-MEM/10%FBS/PenStrepGlu. After determining the number of cells by trypan blue and hemacytometer, 2500 cells are plated in 200 microliters of Expansion media per well on collagen I coated 96-well plates (Becton Dickinson Labware, cat # 354407).

An exemplary cell culture procedure is as follows. The starting day for plating the cells is indicated to be a Wednesday. If a different day of the week is used as the starting day for plating the cells, that day will trigger the daily schedule for removing and adding media during the entire process as indicated below. For example, if the cells are plated on a Tuesday, media should not be removed and added on the first Friday and Saturday, nor on the second Friday and Saturday. With a Tuesday start, the plates would be prepared for the calcium assay on the final Sunday. Cells are plated on a Wednesday at 2500 cells in 200 µl of Expansion media. On Thursday all of the Expansion media is removed and 200 µl of Differentiation Media is added. On Friday 100 µl of media is removed and 100 µl of fresh Differentiation Media is added. On Monday 100 µl of media is removed and 100 µl of fresh Differentiation Media is added. On Tuesday 100 µl of media is removed and 100 µl of fresh Differentiation Media is added. On Wednesday 100 µl of media is removed and 100 µl of fresh Differentiation Media is added. On Thursday 100 µl of media is removed and 100 µl of fresh Differentiation Media is added. On Friday 100 µl of media is removed and 100 µl of fresh Differentiation Media is added. On the following Monday plates are prepared for the calcium assay as follows: Plates are washed once with 10 mM Tris, HCl pH 7-8. Working under a fume hood, 200 µl of 0.5 N HCl is added per well. Plates are then frozen at -80°C. Just prior to measuring calcium, the plates are freeze-thawed twice, and then trituration with a multichannel pipette is used to disperse the contents of the plate. The contents of the plate is then allowed to settle at 4°C for 30 minutes at which point an appropriate amount of supernatant is removed for measuring calcium using a commercially available calcium kit. An exemplary and not-limiting kit is Calcium (CPC) Liquicolor, Cat. No. 0150-250, Stanbio Laboratory, Boerne, TX.

In this cell based assay, sclerostin inhibits one or more of the sequence of events leading up to and including mineral deposition (i.e., sclerostin inhibits mineralization). Thus, in experiments where sclerostin is included in the particular cell culture experiment, the recombinant sclerostin is added to the media starting on the first Thursday and every feeding day thereafter. In cases where an anti-sclerostin antibody is being tested for the ability to neutralize sclerostin, i.e., allow for mineralization by neutralizing sclerostin's ability to inhibit mineralization, the antibody is added to the media starting on the first Thursday and every feeding day thereafter. The antibody is preincubated with the recombinant sclerostin in Differentiation media for 45-60 minutes at 37°C and then this media is used for feeding the cells.

Described above is a 12-day mineralization protocol for MC3T3-E1-BF cells. Mineralization of the original MC3T3-E1 cells is inhibited by recombinant sclerostin and this inhibition is blocked using an anti-sclerostin neutralizing antibody, e.g., an anti-sclerostin antibody comprising CDRs of SEQ ID NO: 245-247 and 78-80. The cell-based neutralization assay is further described in U.S. Patent Publication No. 7,592,429 at, e.g., Example 8.

*Bone specific alkaline phosphatase assay* - An exemplary bone specific alkaline phosphatase assay is described in International Patent Publication No. WO 2008/115732 and U.S. Patent No. 7,744,874. An exemplary protocol is as follows. C2C12 cells (ATCC, CRL 1772) are plated at 3000-5000 cells/well in a 96-well tissue culture plate in MEM medium supplemented with 5% fetal calf serum. The plate is incubated at 37°C in 5% CO₂ overnight. The antibody is diluted in 0.5X Wnt3a-conditioned medium (prepared as described in WO 2008/115732) to various final concentrations. The medium is removed from the plated cells and a pre-mixed antibody- BMP4-sclerostin solution (human or cynomologous monkey) is added (150 µl), providing an antibody final concentration of 30 µg/ml to 0.5 µg/ml, a final BMP-4 concentration of 25 ng/ml, a final sclerostin protein concentration of 1.0 µg/ml, and the conditioned medium is at 0.5X concentration. The plate is then incubated at 37°C in 5% CO₂ for 72 hours. The medium is removed from the cells, which are washed once with PBS, and frozen and thawed three times alternating between -80°C and 37°C. Alkaline phosphatase activity is measured by adding alkaline phosphatase substrate (1-step PNPP, Pierce #37621) (150 µl/well). The plate of cells is incubated for 60 minutes at room temperature, at which time optical density (OD) is measured at 405 nm to determine alkaline phosphatase activity. IC₅₀ calculations may be performed using, e.g., SigmaPlot Regression Wizard with a Sigmoid 4-parameter fit equation.

*BMP2-induced MC3T3 cell mineralization assay* - An exemplary BMP2-induced mineralization assay in MC3T3 cells is described in International Patent Publication No. WO 2009/047356. Briefly, MC3T31b cells are seeded in 96-well plates (e.g., 6 x 10³ cells/well or 2 x 10³ cells/well) in 100 µl assay culture medium (maintenance culture medium without G418) and incubated for three days to reach confluence. The assay culture medium is changed and compounds to be tested are added with 10 mM b-glycerophosphate and 50 µM ascorbic acid. Prior to addition to the cells, sclerostin and a candidate antibody are preincubated on a separate plate for two hours at room temperature. To the assay 96 well-plates, 2.1 or 2.8 nM BMP-2 (R&D Systems, Cat#355-BM-010) is applied before applying the sclerostin-antibody mixture. Cells are incubated for 14 days. At the end of the incubation, cells are washed twice with 200 µl PBS/well, 50 µl of 0.5 M HCl is added to each well, and plates are frozen at -20°C for a minimum of 24 hours. Plates are thawed at room temperature for 2 hours for testing. Ten microliters of each well is transferred to a new plate and exposed to Calcium Working Solution (1:5) (200µl). Optical density is measured after a 5-30 minute incubation period at 595 nm on a microplate reader. Absorbance is translated into microgram of calcium according to a standard curve, allowing determination of the extent of BMP-2-induced mineralization.

*Cell-based wnt signaling assay* - An exemplary cell-based signaling assay employing super top flash (STF) reporter protein is described in International Patent Publication No. WO 2009/047356. HEK293 cells are transfected with pcDNA3+ (480 ng); SuperTopFlash (STF) (20 ng); and phRL-CMV (0.5 ng) for control wells and pcDNA-wnt1 (20 ng); pcDNA3+ (460 ng); SuperTopFlash (STF) (20 ng); and phRL-CMV (0.5 ng) for Wnt1 treatment wells. The plasmids are mixed with 1.6 µl of Lipofectamine 2000 diluted into 50 µl of OptiMEM® and incubated for 30 minutes at room temperature prior to application to the cells. Once applied, the cells are incubated at 37°C in 5% CO₂ for five hours.

Antibodies are premixed with SOST to generate a series of dilutions. One ml of medium for each dilution is prepared, and 450 µl is added to each well after removing transfection mix. The cells are incubated with the antibody-SOST mixtures for 18-20 hours. At the end of the incubation, medium is removed, and 300 µl of IX Passive Lysis Buffer (Promega, Cat#E194A) is added to lyse cells. Luciferase activity is then measured using Dual-Glo Luciferase System (Promega, Cat#E2940) with 30 µl of lysates in duplicates. Typically, 30 µl of Dual-Glo luciferase (firefly luciferase; for STF) and 30 µl of Dual-Glo Stop and Glo (Renilla luciferase; for transfection efficiency control) substrates is used. Luminescent signals are measured with Mithras LB940 instrument (Berthold Technologies). The ratio of firefly to *Renilla* luciferases is calculated. The final results are expressed by setting the value of Wnt1 without SOST as 1. Additional details of the assay are provided in International Patent Publication No. WO 2009/047356.

### Example 2

This example describes *in vivo* studies wherein an anti-sclerostin antibody improved bone mineral density in postmenopausal women.

A multicenter, international, randomized, parallel group study in postmenopausal women with low BMD at lumbar vertebrae, total hip or femoral neck was conducted. 419 subjects were randomized to receive one of five regimens of an anti-sclerostin antibody, placebo, open label alendronate (ALN), or open label teriparatide (TPTD) for 12 months. The placebo group was further randomized to match the schedules corresponding to the five anti-sclerostin antibody regimens. The subject distribution was as follows: 52 subjects to the placebo arm, 51 subjects to ALN arm, 55 subjects to TPTD arm, and 51, 54, 51, 53, and 52 subjects to anti-sclerostin antibody 70 mg QM, 140 mg Q3M, 140 mg QM, 210 mg Q3M, and 210 mg QM arms, respectively. 383 subjects completed the 12 months of study. Among all subjects enrolled in the study, the mean age was 66.8 years old, 86.4% of subjects were Caucasian, and the mean pretreatment baseline lumbar vertebrae and total hip BMD T-scores were -2.29 and -1.53, respectively. The primary analysis occurred after all subjects had the opportunity to complete the month 12 study visit. BMD was measured by DXA. Overall, the proportions of patients reporting adverse events were balanced between placebo arm and anti-sclerostin antibody arms.

### Bone mineral density

*Lumbar vertebrae BMD percent change from pretreatment baseline at month 6:* At month 6, the mean percent change of lumbar vertebrae BMD from pretreatment baseline in the control groups was 0.3% in placebo arm, 2.6% in alendronate arm, and 4.8% in TPTD arm. In the anti-sclerostin antibody arms, the mean percent change of lumbar vertebrae BMD from pretreatment baseline was 4.1% in 70 mg QM arm, 4.2% in 140 mg Q3M arm, 4.4% in 210 mg Q3M arm, 7.1% in 140 mg QM arm, and 8.2% in 210 mg QM arm. Compared to the placebo arm, each of the anti-sclerostin antibody arms significantly increased lumbar vertebrae BMD at month 6 (p-values < 0.0001).

*Lumbar vertebrae BMD percent change from pretreatment baseline at month 12:* At month 12, the mean percent change of lumbar vertebrae BMD from pretreatment baseline in the control groups was -0.1% in placebo arm, 4.1% in alendronate arm, and 7.1% in TPTD arm. In the anti-sclerostin antibody arms, the mean percent change of lumbar vertebrae BMD from pretreatment baseline was 5.4% in 70 mg QM arm, 5.4% in 140 mg Q3M arm, 5.5% in 210 mg Q3M arm, 9.1% in 140 mg QM arm, and 11.3% in 210 mg QM arm. Each of the anti-sclerostin antibody arms significantly increased lumbar vertebrae BMD at month 12 (p-values < 0.0001) after adjusting for multiplicity. The mean percent change from pretreatment baseline in lumbar vertebrae BMD by visit and comparisons with the active open-label controls is summarized in Table A.

**TABLE A: Lumbar Vertebrae BMD % Change from Baseline at 12 Months**

| | | | | Ab-5 | | | | |
|---|---|---|---|---|---|---|---|---|
| | Placebo | ALN | TPTD | 70 mg QM | 140 mg Q3M | 140 mg QM | 210 mg Q3M | 210 mg QM |
| Mean | -0.1 | 4.1 | 7.1 | 5.4 | 5.4 | 9.1 | 5.5 | 11.3 |
| Difference from Placebo (Mean) | | | | 5.5 | 5.6 | 9.2 | 5.6 | 11.5 |
| Difference from ALN (Mean) | | | | 1.3 | 1.4 | 5.0 | 1.5 | 7.3 |
| Difference from TPTD (Mean) | | | | -1.8 | -1.7 | 2.0 | -1.6 | 4.2 |

*Total hip BMD percent change from pretreatment baseline at months 12 and 6:* At month 12, the mean percent change of total hip BMD from pretreatment baseline in the control groups were -0.7% in placebo arm, 1.9% in alendronate arm, and 1.3% in TPTD arm. In the anti-sclerostin antibody arms, the mean percent change of total hip BMD from pretreatment baseline was 1.3% in 70 mg QM arm, 1.3% in 140 mg Q3M arm, 1.9% in 210 mg Q3M arm, 3.4% in 140 mg QM arm, and 4.1% in 210 mg QM arm. Compared to the placebo arm, each of the anti-sclerostin antibody arms significantly increased total hip BMD at month 12 (p-values < 0.0001). At month 6, the increase of total hip BMD in each of the anti-sclerostin antibody arms was significantly higher than that in the placebo arm (p-values < 0.0061). The mean percent change from pretreatment baseline in total hip BMD by visit and comparisons with the active open-label controls at 12 months is shown in Table B.

**TABLE B: Total Hip BMD % Change from Baseline at 12 Months**

| | | | | Ab-5 | | | | |
|---|---|---|---|---|---|---|---|---|
| | Placebo | ALN | TPTD | 70 mg QM | 140mg Q3M | 140 mg QM | 210 mg Q3M | 210 mg QM |
| Mean | -0.7 | 1.9 | 1.3 | 1.3 | 1.3 | 3.4 | 1.9 | 4.1 |
| Difference from Placebo (Mean) | | | | 2.0 | 2.1 | 4.1 | 2.6 | 4.9 |
| Difference from ALN (Mean) | | | | -0.7 | -0.6 | 1.5 | 0.0 | 2.2 |
| Difference from TPTD (Mean) | | | | -0.1 | 0.0 | 2.1 | 0.6 | 2.8 |

*Femoral neck BMD percent change from pretreatment baseline at months 12 and 6*: At month 12, the mean percent change of femoral neck BMD from pretreatment baseline in control groups was -1.1% in placebo arm, 1.2% in alendronate arm, and 1.1% in TPTD arm. In the anti-sclerostin antibody arms, the mean percent change of femoral neck BMD from pretreatment baseline was 0.6% in 70 mg QM arm, 1.8% in 140 mg Q3M arm, 1.4% in 210 mg Q3M arm, 4.2% in 140 mg QM arm, and 3.7% in 210 mg QM arm. Compared to the placebo arm, each of the anti-sclerostin antibody arms significantly increased femoral neck BMD at month 12 (p-values < 0.0044). At month 6, the increase of femoral neck BMD in the anti-sclerostin antibody 140 mg QM, 210 mg QM and 210 mg Q3M arms was significantly higher than that in the placebo arm (p-values <= 0.0164). The increase was not significantly different than placebo in the anti-sclerostin antibody 70 mg QM arm and the 140 mg Q3M arm (lowest doses for each schedule). The mean percent change from pretreatment baseline in femoral neck BMD by visit and comparisons with the active open-label controls is summarized in Table C.

**TABLE C: Femoral Neck BMD % Change from Baseline at 12 Months**

| | | | | Ab-5 | | | | |
|---|---|---|---|---|---|---|---|---|
| | Placebo | ALN | TPTD | 70 mg QM | 140 mg Q3M | 140 mg QM | 210 mg Q3M | 210 mg QM |
| Mean | -1.1 | 1.2 | 1.1 | 0.6 | 1.8 | 4.2 | 1.4 | 3.7 |
| Difference from Placebo (Mean) | | | | 1.8 | 2.9 | 5.3 | 2.5 | 4.8 |
| Difference from ALN (Mean) | | | | -0.6 | 0.69 | 3.0 | 0.2 | 2.5 |
| Difference from TPTD (Mean) | | | | -0.5 | 0.6 | 3.1 | 0.3 | 2.6 |

*Distal 1*/*3 radius BMD percent change from pretreatment baseline at month 12:* At month 12, the mean percent change of distal 1/3 radius BMD from pretreatment baseline in control groups was -0.9% in placebo arm, -0.3% in alendronate arm, and -1.7% in TPTD arm. In the anti-sclerostin antibody arms, the mean percent change of femoral neck BMD from pretreatment baseline was -1.8% in 70 mg QM arm, -1.1% in 140 mg Q3M arm, -0.4% in 210 mg Q3M arm, -1.0% in 140 mg QM arm, and -1.2% in 210 mg QM arm. Compared to the placebo arm, there is no significant decrease in the distal 1/3 radius at month 12 in any of the anti-sclerostin antibody arms.

### Bone markers

*Percent change in bone resorption marker (CTX):* For all anti-sclerostin antibody doses, serum CTX initially decreased from pretreatment baseline. The largest median decrease for all anti-sclerostin antibody arms was at week 1 and the higher doses remained below pretreatment baseline for up to 12 months.

*Percent Change in bone formation markers (P1NP, BSAP, and osteocalcin (OC)):* For all anti-sclerostin antibody doses, P1NP, BSAP, and OC increased from pretreatment baseline at week 1, peaked at month 1, and returned to pretreatment baseline between month 2 and month 6. At the end of the month 12 observation, the markers of bone formation decreased to levels at or below pretreatment baseline. Overall, the three markers of bone formation exhibited similar profiles.

This example illustrates the ability of the inventive method to significantly increase BMD at the lumbar vertebrae, total hip, and femoral neck. The two highest monthly doses, 140 mg QM and 210 mg QM, significantly increased BMD at the lumbar vertebrae, total hip, and femoral neck compared with two active comparators, teriparatide (QD) and alendronate (QW). The magnitude of anti-sclerostin antibody-mediated BMD increases followed a dose response pattern.

The decrease in bone resorption associated with anti-sclerostin antibody treatment as assessed with serum CTX was observed at week 1 (earliest measured time point) and for the QM regimens remained below pretreatment baseline during the entire 12 months. Bone formation markers increased early after initiation of anti-sclerostin antibody treatment and decreased over time in a dose dependent fashion, wherein the highest doses were associated with increased markers for a longer period of time. Bone formation marker levels fell to approximately pretreatment baseline levels within two to six months of initiation of treatment. A similar adaptive response was not observed for alendronate or teriparatide. For example, bone formation markers remained elevated over the course of teriparatide treatment. Remarkably, BMD in subjects receiving anti-sclerostin antibody continued to increase despite the normalization of marker levels (i.e., waning of bone formation marker levels from initial peak levels and return of bone resorption levels toward baseline levels). In the 140 mg QM arm, for instance, lumbar vertebrae BMD increased approximately 2% from month six to month twelve of the treatment period while bone formation markers were at approximately pretreatment baseline levels.

## Claims

1. An anti-sclerostin antibody comprising a CDR-H1 of SEQ ID NO:245, a CDR-H2 of SEQ ID NO:246, a CDR-H3 of SEQ ID NO:247, a CDR-L1 of SEQ ID NO:78, a CDR-L2 of SEQ ID NO:79 and a CDR-L3 of SEQ ID NO:80, for use in a method for increasing bone mineral density in a postmenopausal woman, the method comprising administering to a postmenopausal woman suffering from osteoporosis said anti-sclerostin antibody in an amount and for a treatment period effective to increase lumbar vertebrae bone mineral density (BMD) at least 9% from pretreatment baseline at twelve months following initial administration of the anti-sclerostin antibody; wherein the amount of anti-sclerostin antibody administered is 140 mg to 210 mg and the amount of the anti-sclerostin antibody is administered at an interval no more frequent than once a month.

2. The anti-sclerostin antibody for use according to claim 1, wherein the anti-sclerostin antibody is administered in an amount and for a treatment period effective to increase lumbar vertebrae BMD at least one standard deviation from pretreatment baseline at twelve months following initial administration of the anti-sclerostin antibody.

3. The anti-sclerostin antibody for use according to claim 1 or claim 2, wherein the amount of anti-sclerostin antibody is administered at a frequency of once a month for a treatment period of three months to 18 months.

4. The anti-sclerostin antibody for use according to any one of claims 1-3, wherein the amount of anti-sclerostin antibody administered is 210 mg.

5. The anti-sclerostin antibody for use according to any one of claims 1-4, wherein
(i) the amount and the treatment period are effective to reduce the risk of vertebral and/or nonvertebral fractures; and/or
(ii) the amount and the treatment period are effective to increase total hip BMD at least 3% from pretreatment baseline at twelve months following initial administration of the anti-sclerostin antibody.

6. The anti-sclerostin antibody for use according to any one of claims 1-5, wherein the anti-sclerostin antibody is an immunoglobulin comprising heavy chains and light chains.

7. The anti-sclerostin antibody for use according to any one of claims 1-6, wherein the anti-sclerostin antibody is an IgG antibody.

8. The anti-sclerostin antibody for use according to any one of claims 1-7, wherein the anti-sclerostin antibody
(i) demonstrates a binding affinity for sclerostin of SEQ ID NO: 1 of less than or equal to 1 x 10⁻⁷ M;
(ii) is capable of neutralizing human sclerostin in a MC3T3 cell-based mineralization assay when there is less than a 6-fold excess of moles of sclerostin binding sites per well as compared to the number of moles of sclerostin per well;
(iii) has an IC₅₀ of 100 nM or less, 50 nM or less, or 25 nM or less for neutralizing human sclerostin in a cell-based assay, such as a bone specific alkaline phosphatase assay;
(iv) has an IC₅₀ of 100 nM or less for neutralizing human sclerostin in a cell-based Wnt signalling assay in HEK293 cell lines;
(v) has an IC₅₀ of 500 nM or less for neutralizing human sclerostin in a BMP2-induced mineralization assay in MC3T3 cells;
(vi) binds to a sclerostin polypeptide comprising the amino acid sequence set forth in SEQ ID NO: 1, wherein said anti-sclerostin antibody binds to the sequence of SEQ ID NO: 6; or
(vii) cross-blocks the binding of at least one of antibodies Ab-A, Ab-B, Ab-1, Ab-4, Ab-5, Ab-6, Ab-7, Ab-8, Ab-9, Ab-10, Ab-12, Ab-19, Ab-20, Ab-23, and Ab-24 to sclerostin and/or is cross-blocked from binding to sclerostin by at least one of antibodies Ab-A, Ab-B, Ab-1, Ab-4, Ab-5, Ab-6, Ab-7, Ab-8, Ab-9, Ab-10, Ab-12, Ab-19, Ab-20, Ab-23, and Ab-24.

9. The anti-sclerostin antibody for use according to any one of claims 1-8, wherein the anti-sclerostin antibody
(i) comprises heavy chains comprising SEQ ID NO: 378 and light chains comprising SEQ ID NO 376; or
(ii) has heavy chains of SEQ ID NO: 145 or SEQ ID NO: 392 and light chains of SEQ ID NO: 141.

10. The anti-sclerostin antibody for use according to any one of claims 1-9, wherein the anti-sclerostin antibody is
(i) a monoclonal antibody; and/or
(ii) a humanized antibody or a chimeric antibody.

11. The anti-sclerostin antibody for use according to any one of claims 1-10, wherein an anti-resorptive is administered to the human following the treatment period, optionally a treatment period of 12 months.

12. The anti-sclerostin antibody for use according to claim 11 wherein the anti-resorptive is:
(i) a bisphosphonate;
(ii) a RANKL inhibitor;
(iii) a RANKL inhibitor wherein the RANKL inhibitor is an anti-RANKL antibody; or
(iv) a RANKL inhibitor wherein the RANKL inhibitor is the anti-RANKL antibody denosumab.

## Patentansprüche

1. Anti-Sclerostin-Antikörper, umfassend eine CDR-H1 von SEQ ID NO: 245, eine CDR-H2 von SEQ ID NO: 246, eine CDR-H3 von SEQ ID NO: 247, eine CDR-L1 von SEQ ID NO: 78, eine CDR-L2 von SEQ ID NO: 79 und eine CDR-L3 von SEQ ID NO: 80, zur Verwendung in einem Verfahren zum Erhöhen der Knochenmineraldichte (Bone Mineral Density, BMD) in einer postmenopausalen Frau, wobei das Verfahren Verabreichen des Anti-Sclerostin-Antikörpers in einer Menge und für eine Behandlungsdauer, die wirksam sind, um die Knochenmineraldichte der Lendenwirbel um mindestens 9 % vom Ausgangswert vor Behandlungsbeginn nach zwölf Monaten nach Anfangsverabreichung des Anti-Sclerostin-Antikörpers zu erhöhen, an eine postmenopausale Frau, die an Osteoporose leidet, umfasst; wobei die verabreichte Menge an Anti-Sclerostin-Antikörper 140 mg bis 210 mg ist und die Menge des Anti-Sclerostin-Antikörpers in einem Intervall von nicht häufiger als einmal im Monat verabreicht wird.

2. Anti-Sclerostin-Antikörper zur Verwendung nach Anspruch 1, wobei der Anti-Sclerostin-Antikörper in einer Menge und für eine Behandlungsdauer, die wirksam sind, um die BMD der Lendenwirbel um mindestens eine Standardabweichung vom Ausgangswert vor Behandlungsbeginn nach zwölf Monaten nach Anfangsverabreichung des Anti-Sclerostin-Antikörpers zu erhöhen, verabreicht wird.

3. Anti-Sclerostin-Antikörper zur Verwendung nach Anspruch 1 oder Anspruch 2, wobei die Menge an Anti-Sclerostin-Antikörper in einer Häufigkeit von einmal im Monat für eine Behandlungsdauer von drei Monaten bis 18 Monaten verabreicht wird.

4. Anti-Sclerostin-Antikörper zur Verwendung nach einem der Ansprüche 1 bis 3, wobei die verabreichte Menge an Anti-Sclerostin-Antikörper 210 mg ist.

5. Anti-Sclerostin-Antikörper zur Verwendung nach einem der Ansprüche 1 bis 4, wobei
(i) die Menge und die Behandlungsdauer wirksam sind, um das Risiko von Wirbel- und/oder Nicht-Wirbelbrüchen zu verringern; und/oder
(ii) die Menge und die Behandlungsdauer wirksam sind, um die Gesamt-BMD der Hüfte um mindestens 3 % vom Ausgangswert vor Behandlungsbeginn nach zwölf Monaten nach Anfangsverabreichung des Anti-Sclerostin-Antikörpers zu erhöhen.

6. Anti-Sclerostin-Antikörper zur Verwendung nach einem der Ansprüche 1 bis 5, wobei der Anti-Sclerostin-Antikörper ein Immunglobulin, das schwere Ketten und leichte Ketten umfasst, ist.

7. Anti-Sclerostin-Antikörper zur Verwendung nach einem der Ansprüche 1 bis 6, wobei der Anti-Sclerostin-Antikörper ein IgG-Antikörper ist.

8. Anti-Sclerostin-Antikörper zur Verwendung nach einem der Ansprüche 1 bis 7, wobei der Anti-Sclerostin-Antikörper
(i) eine Bindungsaffinität für Sclerostin von SEQ ID NO: 1 von weniger als oder gleich 1 x 10⁻⁷ M aufzeigt;
(ii) in der Lage ist, humanes Sclerostin in einem MC3T3-Zell-basierten Mineralisierungsassay zu neutralisieren, wenn weniger als ein 6-facher Überschuss von Molen von Sclerostin-Bindungsstellen pro Well im Vergleich zur Anzahl von Molen von Sclerostin pro Well vorliegt;
(iii) eine IC₅₀ von 100 nM oder weniger, 50 nM oder weniger oder 25 nM oder weniger zum Neutralisieren von humanem Sclerostin in einem zellbasierten Assay, wie z. B. einem knochenspezifischen alkalischen Phosphatase-Assay, hat;
(iv) eine IC₅₀ von 100 nM oder weniger zum Neutralisieren von humanem Sclerostin in einem zellbasierten Wnt-Signalisierungsassay in HEK293-Zelllinien hat;
(v) eine IC₅₀ von 500 nM oder weniger zum Neutralisieren von humanem Sclerostin in einem BMP2-induzierten Mineralisierungsassay in MC3T3-Zellen hat;
(vi) an ein Sclerostin-Polypeptid, das die Aminosäuresequenz dargelegt in SEQ ID NO: 1 umfasst, bindet, wobei der Anti-Sclerostin-Antikörper an die Sequenz von SEQ ID NO: 6 bindet; oder
(vii) die Bindung von mindestens einem der Antikörper Ab-A, Ab-B, Ab-1, Ab-4, Ab-5, Ab-6, Ab-7, Ab-8, Ab-9, Ab-10, Ab-12, Ab-19, Ab-20, Ab-23 und Ab-24 an Sclerostin kreuzblockiert und/oder von Bindung an Sclerostin durch mindestens einen der Antikörper Ab-A, Ab-B, Ab-1, Ab-4, Ab-5, Ab-6, Ab-7, Ab-8, Ab-9, Ab-10, Ab-12, Ab-19, Ab-20, Ab-23 und Ab-24 kreuzblockiert ist.

9. Anti-Sclerostin-Antikörper zur Verwendung nach einem der Ansprüche 1 bis 8, wobei der Anti-Sclerostin-Antikörper
(i) schwere Ketten, die SEQ ID NO: 378 umfassen, und leichte Ketten, die SEQ ID NO: 376 umfassen, umfasst; oder
(ii) schwere Ketten von SEQ ID NO: 145 oder SEQ ID NO: 392 und leichte Ketten von SEQ ID NO: 141 hat.

10. Anti-Sclerostin-Antikörper zur Verwendung nach einem der Ansprüche 1 bis 9, wobei der Anti-Sclerostin-Antikörper
(i) ein monoklonaler Antikörper; und/oder
(ii) ein humanisierter Antikörper oder ein chimärer Antikörper ist.

11. Anti-Sclerostin-Antikörper zur Verwendung nach einem der Ansprüche 1 bis 10, wobei dem Menschen nach der Behandlungsdauer, optional einer Behandlungsdauer von 12 Monaten, ein antiresorptives Mittel verabreicht wird.

12. Anti-Sclerostin-Antikörper zur Verwendung nach Anspruch 11, wobei das antiresorptive Mittel Folgendes ist:
(i) ein Bisphosphonat;
(ii) ein RANKL-Inhibitor;
(iii) ein RANKL-Inhibitor, wobei der RANKL-Inhibitor ein Anti-RANKL-Antikörper ist; oder
(iv) ein RANKL-Inhibitor, wobei der RANKL-Inhibitor der Anti-RANKL-Antikörper Denosumab ist.

## Revendications

1. Anticorps anti-sclérostine comprenant un CDR-H1 de la SEQ ID n° : 245, un CDR-H2 de la SEQ ID n° : 246, un CDR-H3 de la SEQ ID n° : 247, un CDR-L1 de la SEQ ID n° : 78, un CDR-L2 de la SEQ ID n° : 79 et un CDR-L3 de la SEQ ID n° : 80, en vue d'une utilisation dans une méthode d'augmentation de la densité minérale osseuse chez une femme ménopausée, la méthode comprenant l'administration à une femme ménopausée souffrant d'ostéoporose dudit anticorps anti-sclérostine selon une quantité et pendant une période de traitement efficaces pour augmenter une densité minérale osseuse (DMO) de vertèbres lombaires d'au moins 9 % à partir d'une référence de pré-traitement à douze mois suivant une administration initiale de l'anticorps anti-sclérostine, la quantité d'anticorps anti-sclérostine administrée allant de 140 mg à 210 mg et la quantité d'anticorps anti-sclérostine étant administrée à une fréquence de pas plus qu'une fois par mois.

2. Anticorps anti-sclérostine destiné à une utilisation selon la revendication 1, l'anticorps anti-sclérostine étant administré dans une quantité et pendant une période de traitement efficaces pour augmenter la DMO de vertèbres lombaires d'au moins un écart standard par rapport à une référence de pré-traitement à douze mois suivant une administration initiale de l'anticorps anti-sclérostine.

3. Anticorps anti-sclérostine destiné à une utilisation selon la revendication 1 ou la revendication 2, dans lequel la quantité d'anticorps anti-sclérostine est administrée à une fréquence d'une fois par mois pendant une période de traitement de trois mois à 18 mois.

4. Anticorps anti-sclérostine destiné à une utilisation selon l'une quelconque des revendications 1 à 3, dans lequel la quantité d'anticorps anti-sclérostine administrée est de 210 mg.

5. Anticorps anti-sclérostine destiné à une utilisation selon l'une quelconque des revendications 1 à 4, dans lequel
(i) la quantité et la période de traitement sont efficaces pour réduire le risque de fractures vertébrales et/ou non vertébrales ; et/ou
(ii) la quantité et la période de traitement sont efficaces pour augmenter la DMO du fémur total d'au moins 3 % par rapport à une référence de pré-traitement à douze mois suivant une administration initiale de l'anticorps anti-sclérostine.

6. Anticorps anti-sclérostine destiné à une utilisation selon l'une quelconque des revendications 1 à 5, l'anticorps anti-sclérostine étant une immunoglobuline comprenant des chaînes lourdes et des chaînes légères.

7. Anticorps anti-sclérostine destiné à une utilisation selon l'une quelconque des revendications 1 à 6, l'anticorps anti-sclérostine étant un anticorps IgG.

8. Anticorps anti-sclérostine destiné à une utilisation selon l'une quelconque des revendications 1 à 7, l'anticorps anti-sclérostine
(i) démontrant une affinité de liaison pour la sclérostine de la SEQ ID n° : 1 inférieure ou égale à 1 x 10⁻⁷ M ;
(ii) étant capable de neutraliser une sclérostine humaine dans un dosage de minéralisation à base de cellules MC3T3 lorsqu'il y a moins de 6 fois plus de moles de sites de liaison de la sclérostine par puits par rapport au nombre de moles de sclérostine par puits ;
(iii) comportant un IC₅₀ de 100 nM ou moins, 50 nM ou moins, ou 25 nM ou moins pour neutraliser une sclérostine humaine dans un dosage à base de cellules, tel qu'un dosage de phosphatase alcaline osseuse spécifique ;
(iv) comportant un IC₅₀ de 100 nM ou moins pour neutraliser une sclérostine humaine dans un dosage de la signalisation Wnt à base de cellules dans des lignées cellulaires de HEK293 ;
(v) comportant un IC₅₀ de 500 nM ou moins pour neutraliser une sclérostine humaine dans un dosage de minéralisation induit par BMP2 dans des cellules MC3T3 ;
(vi) se liant à un polypeptide de sclérostine comprenant la séquence d'acides aminés indiquée dans la SEQ ID n° : 1, ledit anticorps anti-sclérostine se liant à la SEQ ID n° : 6 ; ou
(vii) empêchant, en réaction croisée, la liaison d'au moins un des anticorps Ab-A, Ab-B, Ab-1, Ab-4, Ab-5, Ab-6, Ab-7, Ab-8, Ab-9, Ab-10, Ab-12, Ab-19, Ab-20, Ab-23 et Ab-24 à la sclérostine et/ou empêchant, en réaction croisée, la liaison à la sclérostine par au moins un des anticorps Ab-A, Ab-B, Ab-1, Ab-4, Ab-5, Ab-6, Ab-7, Ab-8, Ab-9, Ab-10, Ab-12, Ab-19, Ab-20, Ab-23 et Ab-24.

9. Anticorps anti-sclérostine destiné à une utilisation selon l'une quelconque des revendications 1 à 8, dans lequel l'anticorps anti-sclérostine
(i) comprend des chaînes lourdes comprenant la SEQ ID n° : 378 et des chaînes légères comprenant la SEQ ID n° : 376 ; ou
(ii) comporte des chaînes lourdes de la SEQ ID n° : 145 ou de la SEQ ID n° : 392 et des chaînes légères de la SEQ ID n° : 141.

10. Anticorps anti-sclérostine destiné à une utilisation selon l'une quelconque des revendications 1 à 9, l'anticorps anti-sclérostine étant
(i) un anticorps monoclonal ; et/ou
(ii) un anticorps humanisé ou un anticorps chimérique.

11. Anticorps anti-sclérostine destiné à une utilisation selon l'une quelconque des revendications 1 à 10, dans lequel un agent anti-résorption est administré à un humain suivant la période de traitement, éventuellement une période de traitement de 12 mois.

12. Anticorps anti-sclérostine destiné à une utilisation selon la revendication 11 dans lequel l'agent anti-résorption est :
(i) un biphosphonate ;
(ii) un inhibiteur de RANKL ;
(iii) un inhibiteur de RANKL, l'inhibiteur de RANKL étant un anticorps anti-RANKL ; ou
(iv) un inhibiteur de RANKL, l'inhibiteur de RANKL étant l'anticorps anti-RANKL dénosumab.
